# EUROPEAN PATENT SPECIFICATION

(11) **EP 4 041 207 B1**
(45) Date of publication and mention of the grant of the patent: **25.02.2026**
(21) Application number: 20873775.9
(22) Date of filing: 08.10.2020
(51) Int. Cl.: A61K 31/015, A61K 31/01, A61K 31/355, A61K 31/575, A61K 8/31, A61K 8/67, A61K 8/97, A61P 17/16, A61P 17/18, A61P 39/06, A61Q 19/08

(54) **COMPOSITIONS AND METHODS FOR INHIBITING COLLAGEN LOSS**
ZUSAMMENSETZUNGEN UND VERFAHREN ZUM HEMMEN VON COLLAGENVERLUST
COMPOSITIONS ET PROCÉDÉS PERMETTANT D'INHIBER LA PERTE DE COLLAGÈNE

(30) Priority: 10.10.2019 US 201962913220 P
(43) Date of publication of application: 17.08.2022
(73) Proprietor: Lycored Ltd., 84102 Be´er Sheva (IL)
(72) Inventor: OFFER, Tal, 3780800 Givat Ada (IL); SEDLOV, Tanya, 8449723 Beer Sheva (IL); SHARONI, Yoav, 8496500 Omer (IL); LEVY, Joseph, 8496500 Omer (IL); LEVY, Rachel, 8496500 Omer (IL); LINNEWIEL HERMONI, Karin, Tenefly, New Jersey 07670 (US)
(74) Representative: Boult Wade Tennant LLP
(86) International application number: PCT/IL2020/051086
(87) International publication number: WO 2021/070184

(56) References cited:
- EP-B1- 1 107 723
- WO-A1-2014/162313
- WO-A2-01/85182
- WO-A2-2008/004206
- FR-A1- 2 805 742
- JP-A- 2016 011 259
- US-A1- 2002 131 940
- US-A1- 2011 212 040
- US-A1- 2011 212 040
- US-A1- 2012 027 707
- "A new product from IBR For skin as smooth and bright as snow", INTERNET CITATION, 15 October 2008 (2008-10-15), pages 1 - 16, XP002686258, Retrieved from the Internet <URL:http://www.ibrweb.com/newslist1.htm> [retrieved on 20121026]
- QUAN TAIHAO ET AL: "Solar Ultraviolet Irradiation Reduces Collagen in Photoaged Human Skin by Blocking Transforming Growth Factor-[beta] Type II Receptor/Smad Signaling", THE AMERICAN JOURNAL OF PATHOLOGY, vol. 165, no. 3, 1 September 2004 (2004-09-01), US, pages 741 - 751, XP093085690, ISSN: 0002-9440, DOI: 10.1016/S0002-9440(10)63337-8
- DENICHILO MARK O. ET AL: "Peroxidase Enzymes Regulate Collagen Extracellular Matrix Biosynthesis", THE AMERICAN JOURNAL OF PATHOLOGY, vol. 185, no. 5, 1 May 2015 (2015-05-01), US, pages 1372 - 1384, XP093222796, ISSN: 0002-9440, DOI: 10.1016/j.ajpath.2015.01.013
- MELÉNDEZ-MARTÍNEZ ANTONIO J., STINCO CARLA M., MAPELLI-BRAHM PAULA: "Skin Carotenoids in Public Health and Nutricosmetics: The Emerging Roles and Applications of the UV Radiation-Absorbing Colourless Carotenoids Phytoene and Phytofluene", NUTRIENTS, vol. 11, no. 5, 16 May 2019 (2019-05-16), pages 1093, XP055816628
- GRETHER-BECK S., MARINI A., JAENICKE T., STAHL W., KRUTMANN J.: "Molecular evidence that oral supplementation with lycopene or lutein protects human skin against ultraviolet radiation: results from a double blind, placebo-controlled, crossover study", BRITISH JOURNAL OF DERMATOLOGY, vol. 176, 31 May 2017 (2017-05-31), pages 1231 - 1240, XP055816635
- BEZALEL LIKI VON OPPEN-, FISHBEIN DANIT, HAVAS FABIEN, BEN-CHITRIT OLGA, KHAIAT ALAIN: "The photoprotective effects of a food supplement tomato powder rich in phytoene and phytofluene, the colorless carotenoids, apreliminary study", GLOBAL DERMATOLOGY, vol. 2, 31 December 2015 (2015-12-31), pages 178 - 182, XP055816641
- SCHAEFER KATIA: "Colorless Carotenoids for Antiaging Inside and Out", 30 October 2008 (2008-10-30), XP055816644, Retrieved from the Internet <URL:https://www.cosmeticsandtoiletries.com/formulating/function/active/6746617.html>
- SOPHIE BULLIMORE: "Golan Raz: Lycored’s ingestible skin care is more than a pretty face", NBR, 3 October 2019 (2019-10-03), XP055816646, Retrieved from the Internet <URL:https://www.nutraceuticalbusinessreview.com/news/article_page/Golan_Raz_Lycoreds_ingestible_skin_care_is_more_than_a_pretty_face/158669>

## Description

### CROSS-REFERENCE TO RELATED APPLICATION

This application claims the benefit of priority of U.S. Provisional Patent Application No. 62/913,220, titled "COMPOSITIONS AND METHODS FOR INHIBITING COLLAGEN LOSS", filed October 10, 2019.

### FIELD OF THE INVENTION

The invention relates generally to the field of carotenoids, and methods of using the same, such as for treating skin-related conditions, including collagen loss.

### BACKGROUND

Decline in skin biological structure and function caused by aging, attracts huge attention primarily due to the elevation in the population average life span. Fibroblasts are major components of the dermis and are known for producing collagen and elastic fibers. The hallmark of a photo aged skin is solar elastosis, which is probably an end product of extracellular matrix (ECM) degradation. The ECM that supports cells in skin tissues includes collagen, elastic fiber and fibrillin.

Neutrophils have been reported to infiltrate the skin following exposure to erythemogenic doses of ultra-violate B (UVB), is a solar simulating radiation (SSR), and a part of the natural sunlight. Exposure of human skin to a certain threshold of UV, infrared radiation, or heat, leads to neutrophils influx. These neutrophils are packed with potent proteolytic enzymes capable of degrading collagen, and elastic fibers. Neutrophil-derived proteolytic enzymes are involved in the ECM damage observed in several non-dermatological conditions. Furthermore, it was suggested that neutrophils, rather than keratinocytes and fibroblasts, may be the key players in photoaging. It was reported that following exposure of white skin to erythemogenic doses of SSR, infiltrating neutrophils and not keratinocytes or fibroblasts were the major sources of proteolytic enzymes (matrix metalloproteases (MMPs) and elastase, in particular). Oxygen-derived metabolites induce activation of proteolytic enzymes, and/or prevent these enzymes from being inactivated by antiproteinases, therefore induce ECM damage which may eventually result in solar elastosis.

WO01/85182 discloses a composition comprising a cartilage extract, hydrophilic antioxidants and lipophilic antioxidants, wherein the lipophilic antioxidant may comprise lycopene, tocopherols, beta-carotene, phytoene and phytofluene useful to improve collagen synthesis in the dermis.

There is still a great need for compositions and methods for preventing the release of neutrophil-derived proteolytic enzymes and therefore protect from collagen damage.

### SUMMARY

The present invention is defined by the claims. Any subject-matter falling outside the scope of the claims is provided for information only.

Reference to methods of treatment of the human body by therapy, referred to in this description are to be interpreted as composition and/or compounds for use in said methods of treatment.

According to a first aspect, there is provided a composition comprising: phytoene in the amount of 55-65% (w/w) of the total carotenoids in said composition, phytofluene in the amount of 10-20% (w/w) of the total carotenoids in said composition, zeta carotene in the amount of 15-25% (w/w) of the total carotenoids in said composition, tocopherol in the amount of 10-30% (w/w) of the total carotenoids in said composition, and an acceptable carrier.

According to another aspect, there is provided a method for preventing or treating collagen loss in a subject in need thereof, comprising administering to the subject a therapeutically effective amount of the herein disclosed composition, thereby preventing or treating collagen-loss in the subject.

According to another aspect, there is provided a method for reducing the amount, the activity, or both, of any one of: a matrix metalloprotease (MMP), myeloperoxidase (MPO), superoxide (SO), elastase, nitric oxide (NO), and any combination thereof, in subject in need thereof, comprising administering the subject with a therapeutically effective amount of the herein disclosed composition.

In some embodiments, the weight ratio of the phytoene and the phytofluene combined to the zeta carotene ranges from 15:1 (w/w) to 2:1 (w/w).

In some embodiments, the composition further comprises an additional carotenoid selected from the group consisting of: lycopene, beta carotene, gamma carotene, and any combination thereof.

In some embodiments, the lycopene is in the amount of less than 5% (w/w) of the total carotenoids in the composition, the beta carotene is in the amount of less than 5% (w/w) of the total carotenoids in the composition, the gamma carotene is in the amount of 0.2-1.5% (w/w) of the total carotenoids in the composition, or a combination thereof.

In some embodiments, the composition comprises total carotenoids amount of 10-15% (w/w) of the composition.

In some embodiments, the composition further comprises a phytosterol.

In some embodiments, the phytosterol is in the amount of 5-15% (w/w) total carotenoids in of the composition.

In some embodiments, the composition is for use in reducing collagen loss, increasing collagen levels, or both.

In some embodiments, preventing or treating is reducing collagen loss by 50-90%.

In some embodiments, the MMP is selected from: MMP-9, MMP-8, MMP-1, or any combination thereof.

In some embodiments, the subject is afflicted with a collagen-loss related disease.

In some embodiments, the collagen-loss related disease is selected from the group consisting of: an age-related disease, a skin disease, and an inflammatory disease.

In some embodiments, the skin disease comprises skin damage induced by any one of: radiation, oxidative stress, DNA damage, telomere shortening, inflammation, tobacco usage, and any combination thereof.

In some embodiments, the radiation comprises UV radiation, visible light radiation, infrared radiation, or any combination thereof.

In some embodiments, the UV radiation is UVA, UVB, UVC, or any combination thereof.

In some embodiments, the subject has increased amount, increased activity, or both, of any one of: MMP, MPO, SO, elastase, NO, and any combination thereof, in the skin, systemically, or both, compared to control.

In some embodiments, administering comprises orally administering, topically administering, or both.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE FIGURES

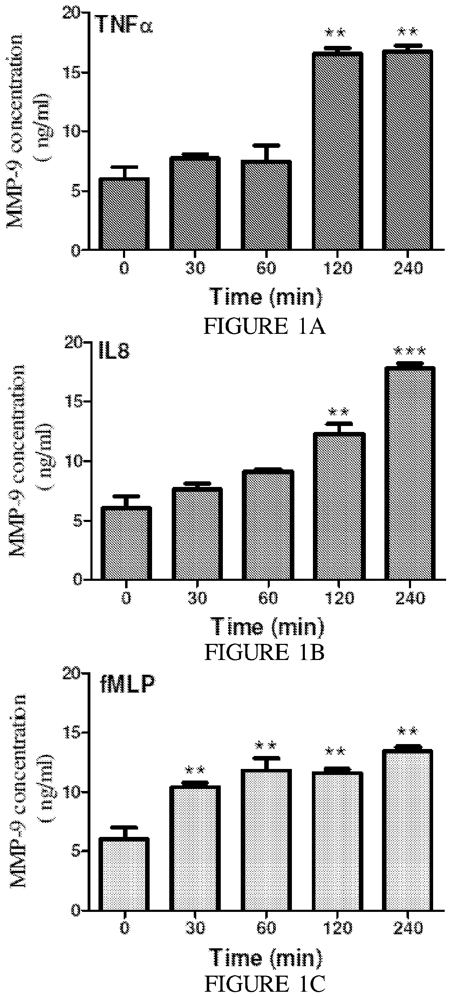

**Figs. 1A-1C** are vertical bar graphs showing the kinetics of matrix metalloprotease 9 (MMP-9) secretion from human neutrophils stimulated by tumor necrosis factor α (TNFα, **1A);** interleukin 8 (IL8, **1B),** and formyl-Methionine-Leucine-Phenylalanine (fMLP, **1C)** agonists. Values represent average ± standard error of the mean (SEM) of 5 independent experiments.

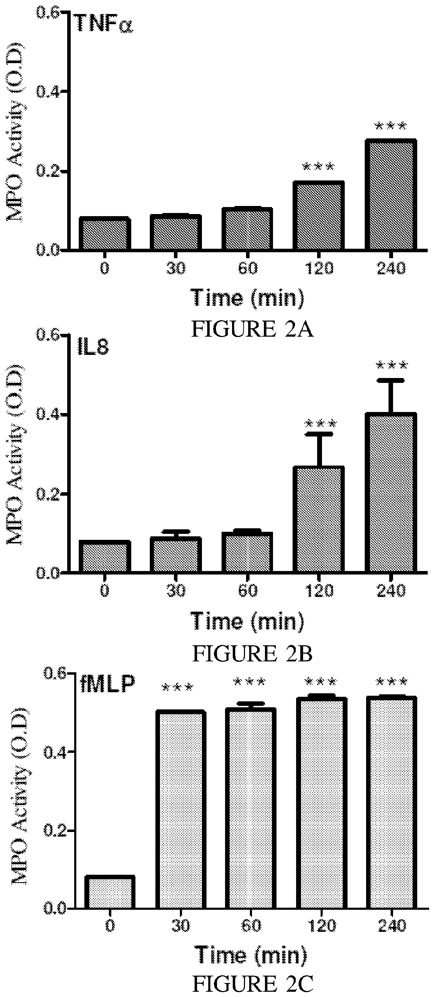

**Figs. 2A-2C** are vertical bar graphs showing the kinetics of myeloperoxidase (MPO) secretion from human neutrophils stimulated by TNFα **(2A);** IL8 **(2B),** and fMLP **(2C)** agonists. Values represent average ± SEM of 5 independent experiments.

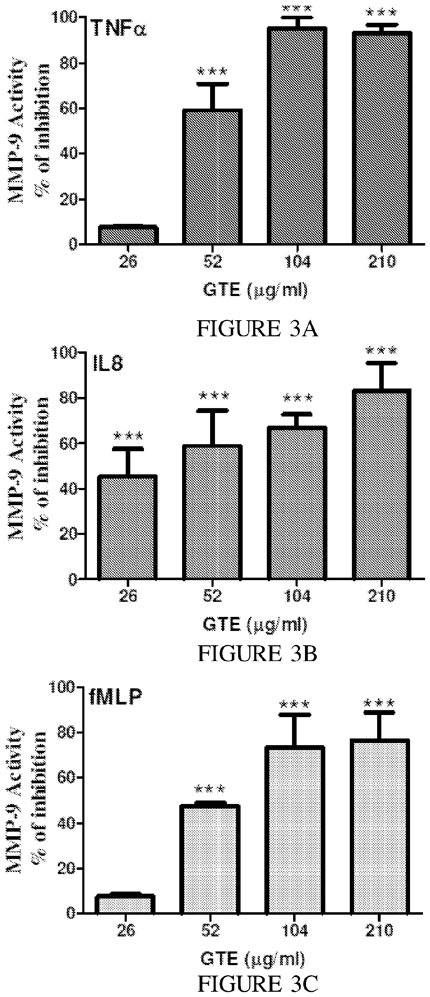

**Figs. 3A-3C** are vertical bar graph showing the inhibitory effect of Lumenato (golden tomato supercritical extract or GTE) on MMP-9 secretion. Human neutrophils were stimulated by TNFα **(3A);** IL8 **(3B),** and fMLP **(3C)** agonists, and a dose dependent inhibition of MMP-9 secretion was observed in the presence of GTE. Values represent average ± SEM of 3 independent experiments.

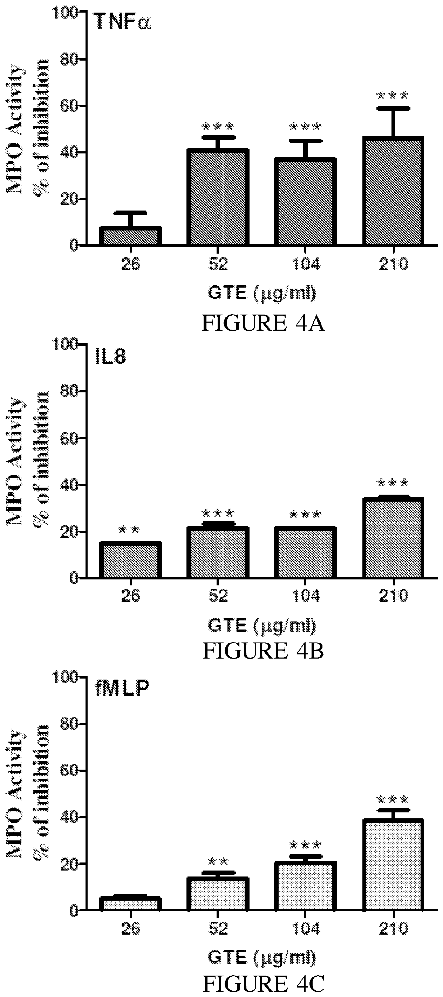

**Figs. 4A-4C** are vertical bar graph showing the inhibitory effect of GTE on MPO secretion. Human neutrophils were stimulated by TNFα **(4A);** IL8 **(4B),** and fMLP **(4C)** agonists, and a dose dependent inhibition of MPO secretion was observed in the presence of GTE. Values represent average ± SEM of 3 independent experiments.

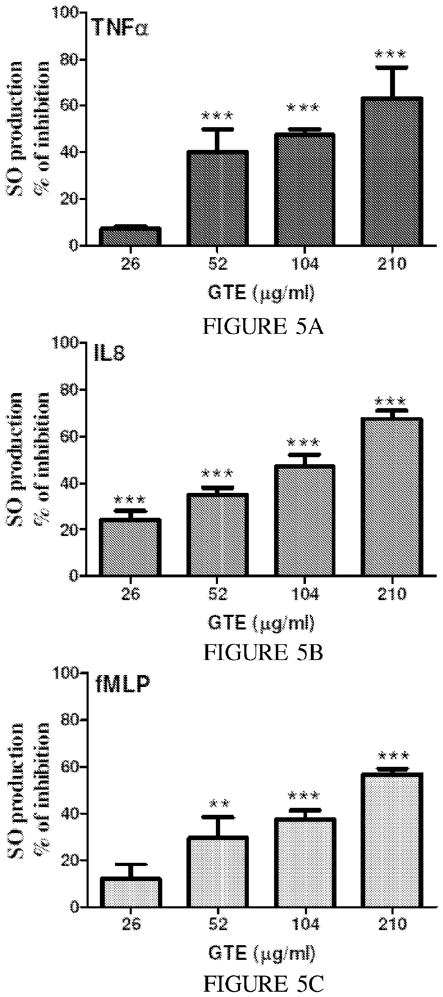

**Figs. 5A-5C** are vertical bar graph showing the inhibitory effect of GTE on superoxide secretion. Human neutrophils were stimulated by TNFα **(5A);** IL8 **(5B),** and fMLP **(5C)** agonists, and a dose dependent inhibition of superoxide secretion was observed in the presence of GTE. Values represent average ± SEM of 3 independent experiments.

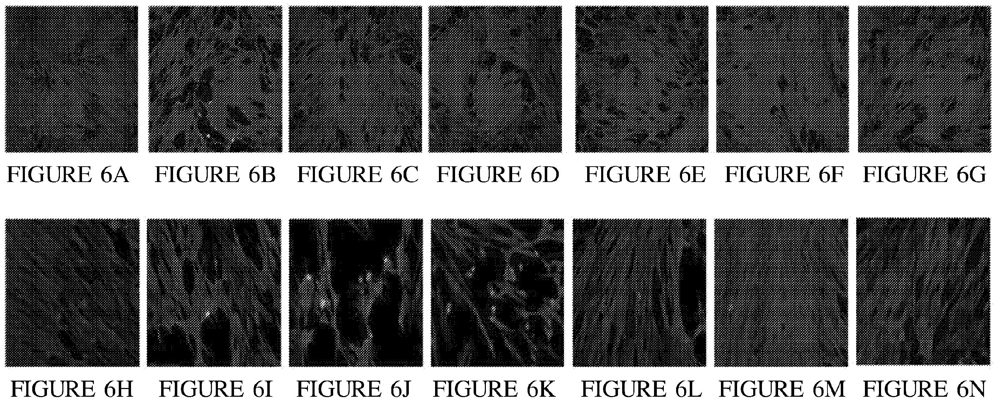

**Figs. 6A-6S** are immunofluorescent micrographs and vertical bar graphs showing that addition of GTE prevented collagen-3 damage in normal human dermal fibroblasts (NHDF) induced by neutrophils, in a dose dependent manner. Neutrophils were stimulated with 100 ng/ml TNFα, and incubated in the presence of gradually increased GTE concentrations: 6.5 µg/ml **(6C, 6J),** 13 µg/ml **(6D, 6K),** 26 µg/ml **(6E, 6L),** 52 µg/ml **(6F, 6M),** and 104 µg/ml **(6G, 6N).** Controls: Negative (no TNFα; **6A** and **6H**), positive (no GTE; **6B** and **6I**). **(6A-6G)** are representative immunofluorescence staining of collagen-3 magnified ×40. **(6H-6N)** are representative immunofluorescence staining of collagen-3 magnified ×200. **(6A-6N)** In each experiment 10 fields of each treatment were scanned. **(6O)** is a vertical bar graph showing the prevention of collagen damage by GTE as analyzed by densitometry of immunofluorescence staining of collagen-3. Values represent the average of 5 different experiments. **(6P)** and **(6Q)** are vertical bar graphs showing the effect of gradually increased concentrations of LycoDerm and LycoMato on prevention of collagen loss, respectively. Higher concentrations of either LycoDerm or LycoMato were toxic to the cells. In contrast, no cellular toxic effect was observed for GTE at 104 µg/ml (the highest concentration that was used). **(6R)** a graph showing densitometry of the immunofluorescence staining of collagen-3 **(6A-6N).** Shown are the mean ± SEM of three different experiments. **(6S)** is a graph showing the amount of pro-collagen-3 secreted to the supernatant of the co-cultures. Shown are the mean ± SEM of three different experiments each in triplicates. The horizontal dashed line indicates the effect of Lumenato without the effect of neutrophils. Lum - Lumenato (µg/ml), Fib - fibroblasts, Neu - neutrophils.

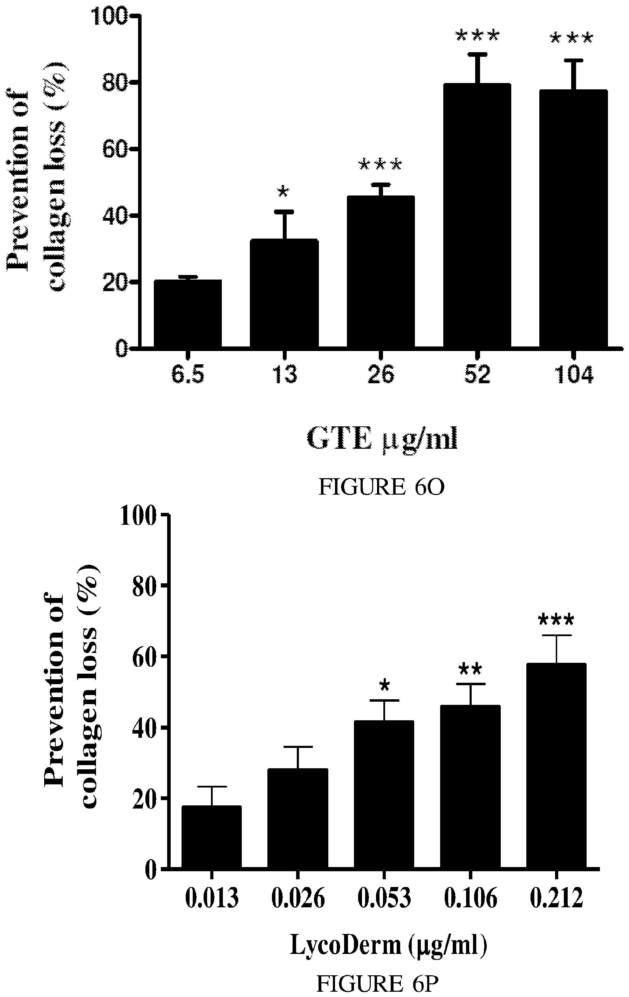

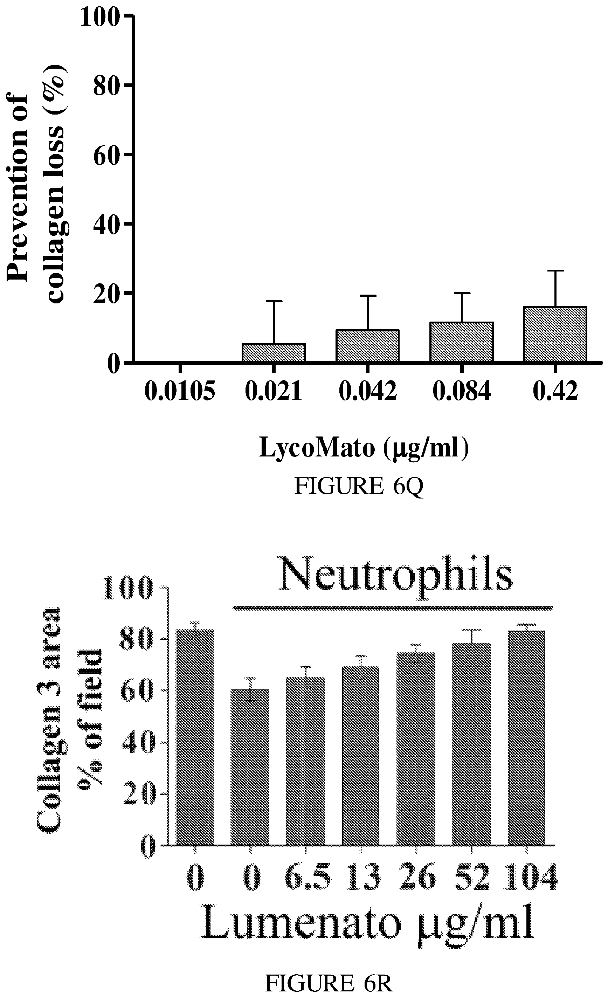

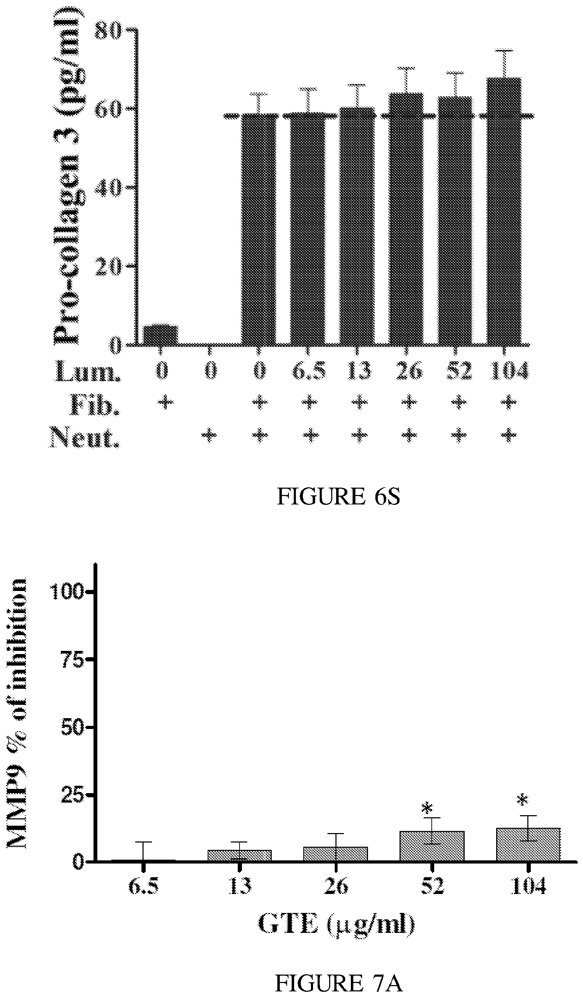

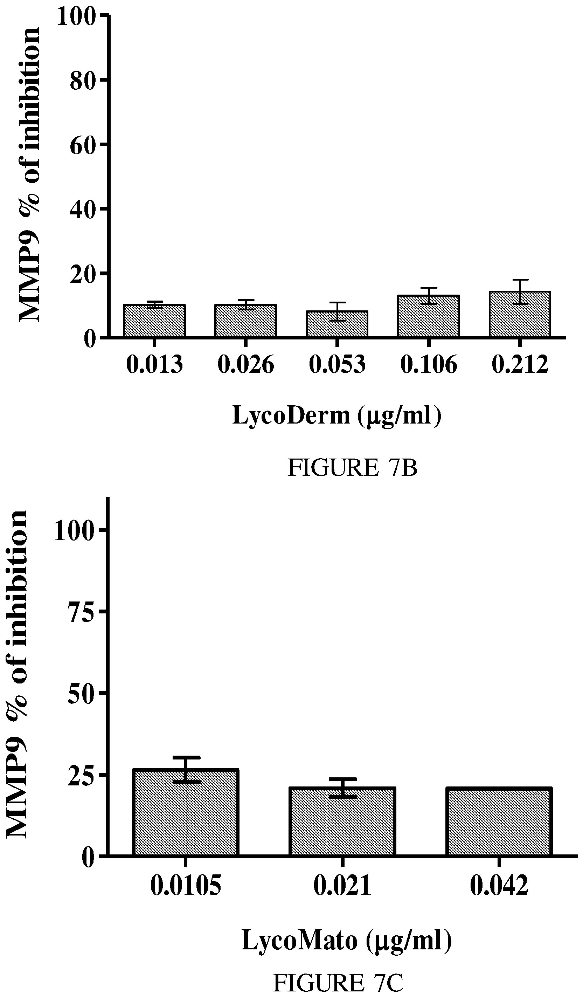

**Figs. 7A-7C** are vertical bar graphs showing that GTE induced inhibition of MMP-9 release from neutrophils cocultured with NHDF (as described in **Fig. 6****).** MMP-9 was quantified in the supernatants of the cocultures **(7A).** Values represent the average of 5 different experiments. **(7B)** and **(7C)** are vertical bar graphs showing the effect of gradually increased concentrations of LycoDerm and LycoMato on MMP-9 levels, respectively. Higher concentrations of either LycoDerm or LycoMato were toxic to the cells. In contrast, no cellular toxic effect was observed for GTE at 104 µg/ml (the highest concentration that was used).

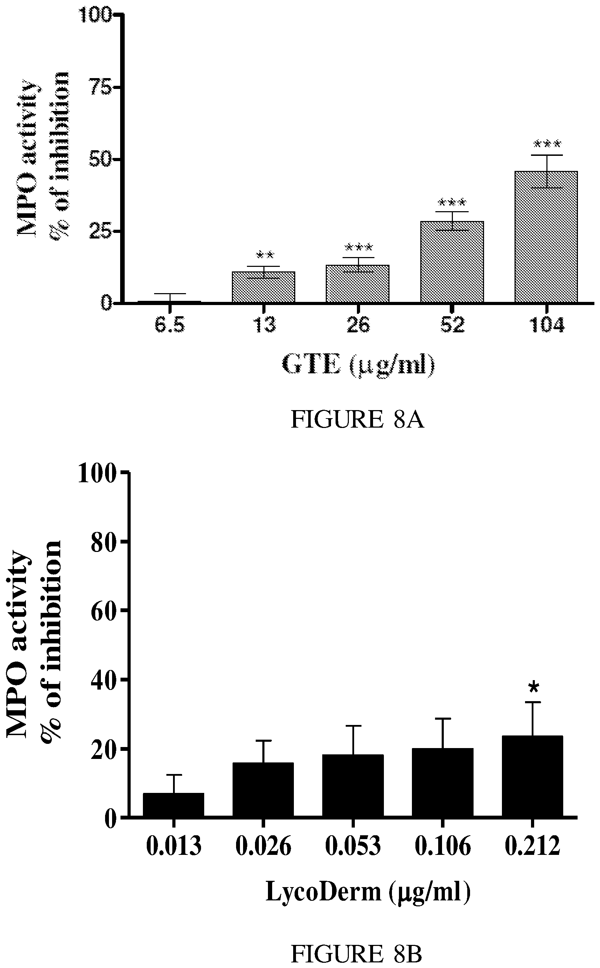

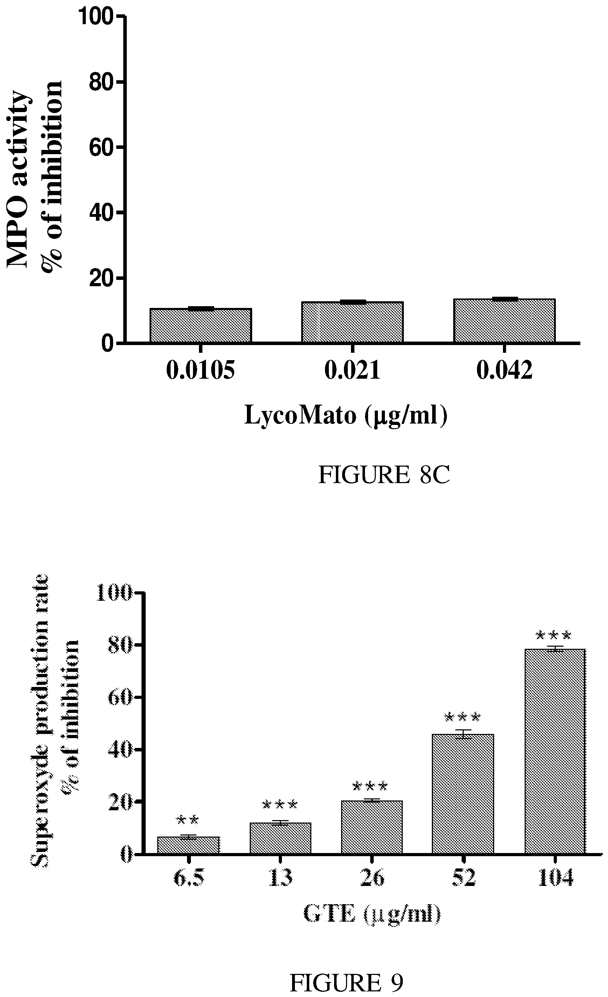

**Figs. 8A-8C** are vertical graphs showing that GTE induced inhibition of MPO release from neutrophils cocultured with NHDF (as described in **Fig. 6****).** MPO quantification was determined based on its activity in the supernatants of the cocultures **(8A).** Values represent the average of 5 different experiments. **(8B)** and **(8C)** are vertical bar graphs showing the effect of gradually increased concentrations of LycoDerm and LycoMato on MPO activity, respectively. Higher concentrations of either LycoDerm or LycoMato were toxic to the cells. In contrast, no cellular toxic effect was observed for GTE at 104 µg/ml (the highest concentration that was used).
**Fig. 9** is a vertical bar graph showing that GTE induced inhibition of superoxide release from neutrophils cocultured with NHDF (as described in **Fig. 6****).** Superoxide levels were detected using Amplex red. Values represent the average of 5 different experiments.

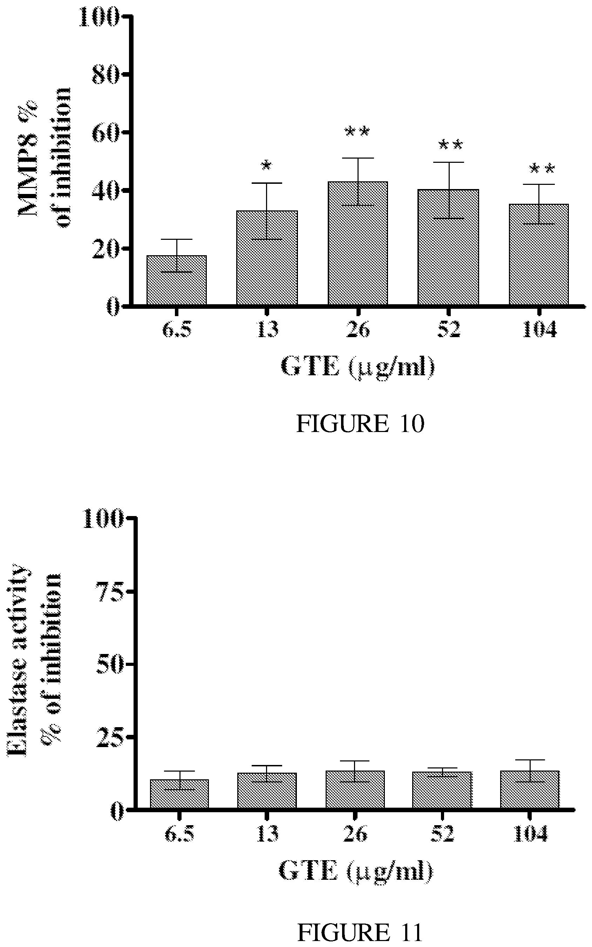

**Fig. 10** is a vertical bar graph showing that GTE induced inhibition of MMP-8 (Collagenase) release from neutrophils cocultured with NHDF (as described in **Fig. 6****).** MMP-8 was quantified in the supernatants of the cocultures. Values represent the average of 5 different experiments.
**Fig. 11** is a vertical bar graph showing that GTE induced inhibition of elastase release from neutrophils cocultured with NHDF (as described in **Fig. 6****).** Elastase quantification was determined based on its activity in the supernatants of the cocultures. Values represent the average of 5 different experiments.

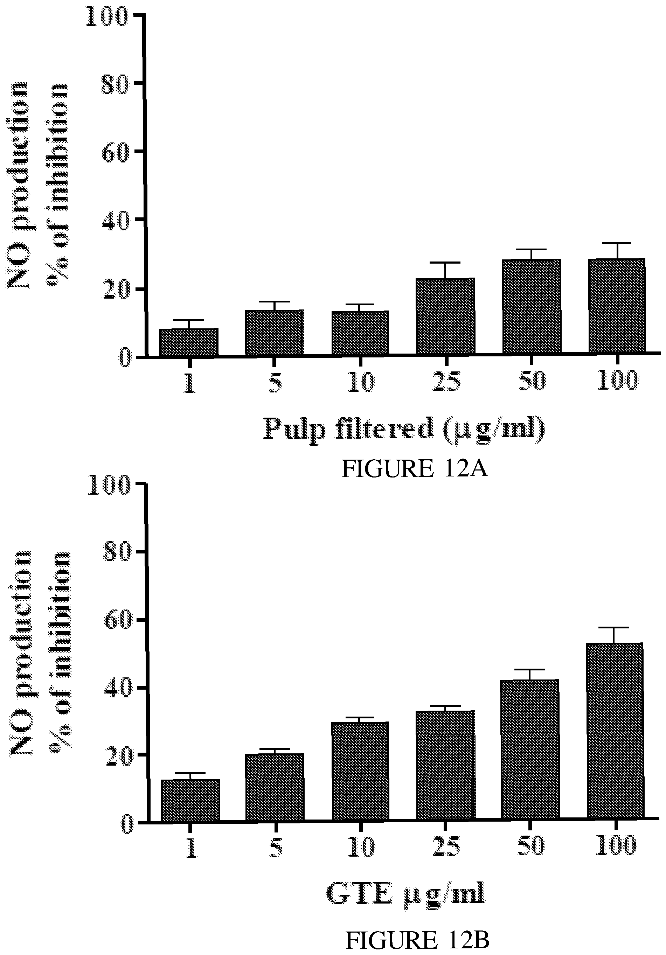

**Figs. 12A-12B** are vertical bar graphs showing the effect of pulp or GTE on nitric oxide (NO) release from macrophages stimulated by lipopolysaccharides (LPS). Pulp was dissolved in medium and filtrated; GTE was dissolved in DMSO. In all concentrations tested there was no effect on cell survival, as determined by MTT reduction. **(12A)** shows the effect of filtered pulp, and **(12B)** shows the effect of GTE on NO production. Results represent the average of 2 experiments performed.

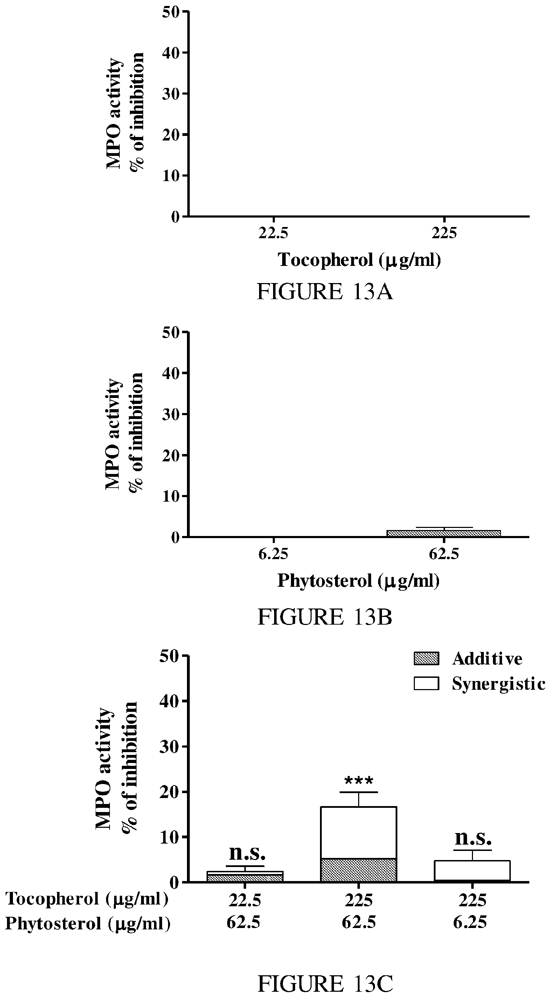

**Figs. 13A-13C** are vertical bar graphs showing that the addition of tocopherol and phytosterol to stimulated neutrophils in the ratio present in GTE resulted in a significant synergistic inhibition of MPO release and activity from neutrophils activated with TNFα. The sole addition of tocopherol **(13A)** or phytosterol **(13B)** had no effect on the release and activity of MPO from activated neutrophils with TNFα. In contrast, 225 µg/ml of Tocopherol and 62.5 µg/ml of Phytosterol, resulted in a synergistic effect of approximately 20% inhibition of MPO activity while a 10-fold higher or lower ratio did not provide a synergistic effect **(13C).**

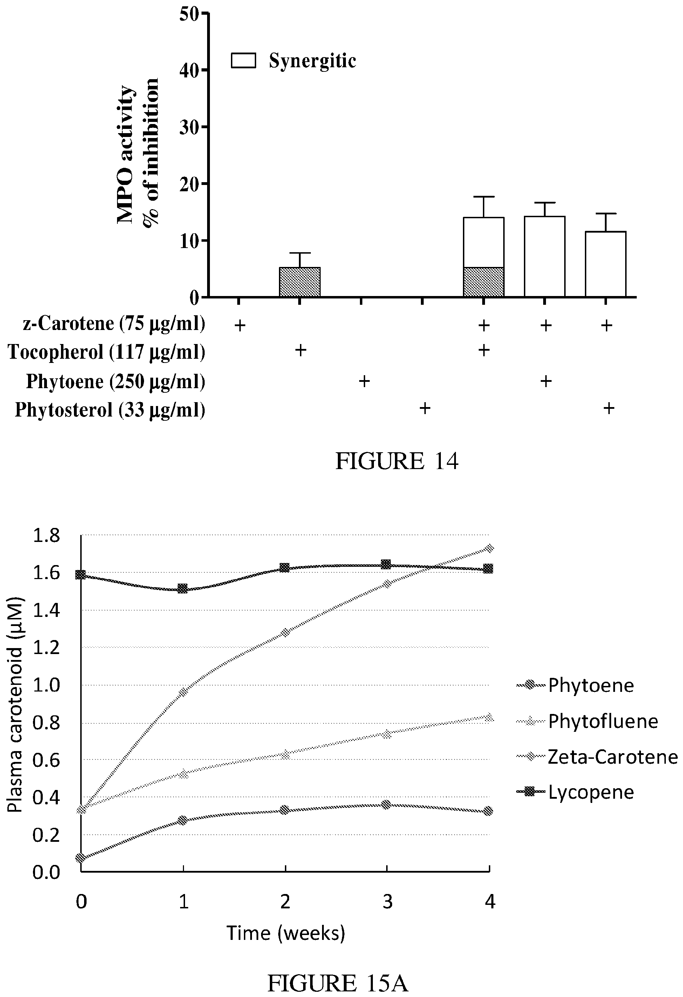

**Fig. 14** is a vertical bar graph showing that the addition of zeta carotene with either phytoene, Tocopherol, or phytosterol, to stimulated neutrophils, in the ratio present in GTE resulted in a significant synergistic inhibition of MPO activity. The sole addition of zeta carotene, Tocopherol, phytoene, or phytosterol had 5% inhibition of MPO activity, at most, or no effect in most cases. The addition of zeta carotene with any one of Tocopherol, phytoene, or phytosterol in the ratio present in GTE induced a synergistic inhibition of MPO activity or release from TNFα-activated neutrophils.
**Figs. 15A-15B** are graphs showing that GTE (e.g., Lumenato) capsules have increased carotenoid bioavailability. Phytoene, phytofluene and zeta-carotene which are the main carotenoids in the golden tomato extract shown to be well absorbed. Zeta carotene was absorbed extremely well as reflected by a steep increase in plasma concentration **(15A). (15B)** is a graph showing the bioavailability kinetics of phytoene and phytofluene only.

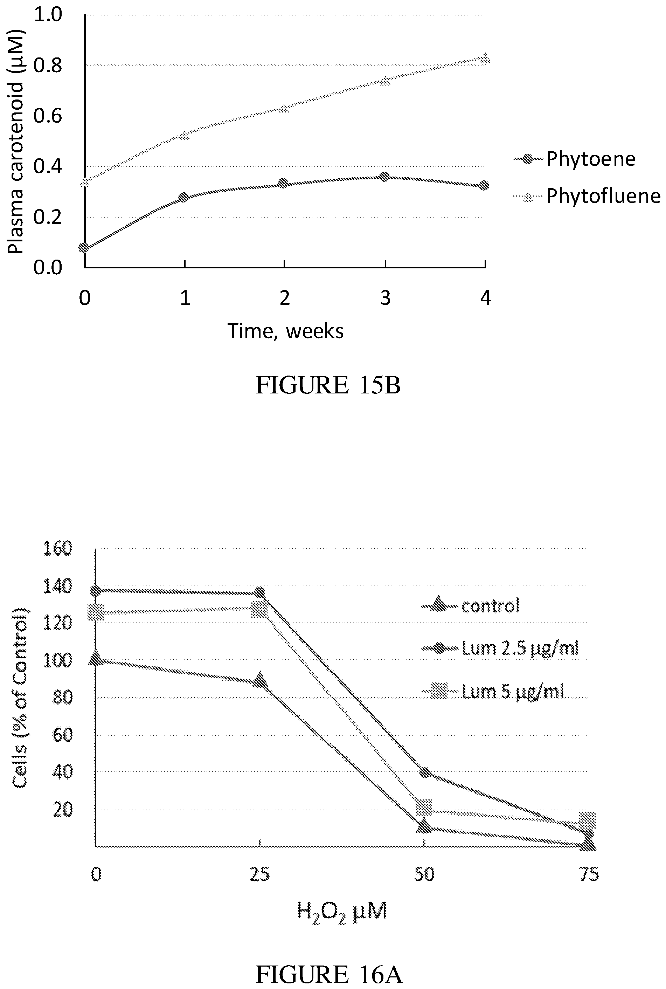

**Figs. 16A-16B** are graphs showing that H₂O₂ induces cell death and increases secretion of MMP1 in a dose dependent manner. The addition of Lumenato at the indicated concentrations before adding H₂O₂ increased cell viability **(16A)** and reduced the secretion of MMP1 **(16B).**

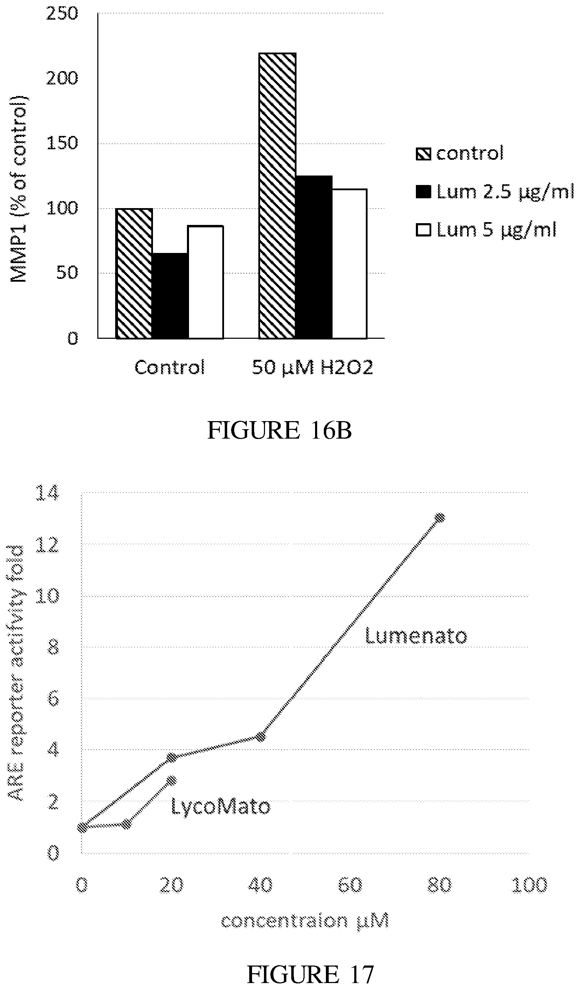

**Fig. 17** is a graph showing that GTE (e.g., Lumenato) increased activation of ARE/Nrf2 transcriptional activity in a dose dependent manner. Lycomato is shown as a reference. The concentrations shown are for: lycopene (in the case of Lycomato), and phytoene (in the case of Lumenato).

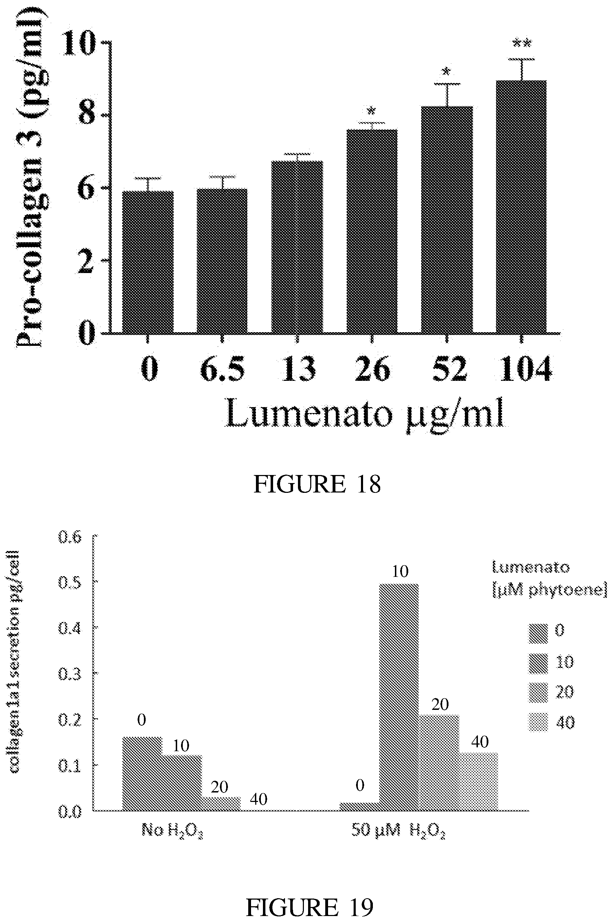

**Fig. 18** includes a vertical bar graph showing levels of pro-collagen-3 in the supernatant of co-cultures. Shown are the mean ± SEM of four different experiments each in triplicates. Lumenato in the range of 6.5-104 µg/ml was added to fibroblasts plated in two different concentrations.
**Fig. 19** incudes a graph showing the Lumenato corrects H₂O₂ induced reduction in collagen 1a1 secretion.

### DETAILED DESCRIPTION

In some embodiments, the present invention is directed to a composition comprising a plurality of carotenoids. The present invention is based, in part, on the surprising findings that a tomato extract comprising high amounts of phytoene, phytofluene, and zeta carotene, prevented collagen loss, and inhibited matrix metalloprotease 9 (MMP-9), and myeloperoxidase (MPO) activity, at greater levels compared to other tomato extracts, and with reduced cellular toxicity compared to the same tomato extracts.

In some embodiments, the composition comprises phytoene, phytofluene, zeta-carotene, and tocopherol. In one embodiment, the composition comprises phytoene, phytofluene, zeta carotene, and an additional carotenoid. In one embodiment, the composition comprises phytoene, phytofluene, zeta carotene, lycopene, beta carotene, gamma carotene, tocopherol, and phytosterol.

In some embodiments, a carotenoid is a natural carotenoid extracted, isolated or purified from a fruit, a vegetable, or a plant (including a plant part). In another embodiment, a carotenoid is carotenoid extracted from a tomato plant. In another embodiment, a carotenoid is a carotenoid extracted from a tomato fruit. In another embodiment, a tomato carotenoid is a tomato extract enriched for a carotenoid. In another embodiment, tomato carotenoid is a carotenoid-rich tomato extract which is all-natural. In another embodiment, tomato carotenoid is a tomato carotenoid complex. In another embodiment, tomato carotenoid complex comprises a complex of phytonutrients including a plurality of carotenoids (such as phytoene, phytofluene, zeta carotene, beta-carotene, etc.), tocopherols and phytosterols. In some embodiments, a carotenoid is a synthetic carotenoid.

In some embodiments, the present invention provides a tomato extract obtained by an innovative extraction protocol. This particular extract which comprises phytoene, phytofluene, and zeta carotene (in amounts as specified hereinbelow), has reduced cellular toxicity. In some embodiments, reduced cellular toxicity is compared to other tomato extracts. In some embodiments, reduced toxicity enables to provide the composition of the invention to a subject in need, at a higher dose without reducing the survival, wellbeing, or both, of the subject. In some embodiments, administering the composition of the invention to a subject in need enables to increase the efficacy of the treatment by providing the active ingredients, such as phytoene, phytofluene, zeta-carotene, and tocopherol at higher amounts which increase the therapeutic effect, but without reducing the survival, wellbeing, or both, of the subject due to high cellular toxicity.

In some embodiments, the composition of the invention provides greater amounts of carotenoids with reduced toxicity compared to other plant-, fruit-, or vegetable-derived extracts, such as a tomato. In some embodiments, the composition of the invention provides increased therapeutic efficacy with reduced toxicity compared to other plant-, fruit-, or vegetable-derived extracts, such as a tomato.

In some embodiments, the composition of the invention comprises natural carotenoids, synthetic carotenoids, or any combination thereof.

In some embodiments, the composition comprises phytoene in the amount of 10-40% (w/w), 15-35% (w/w), 20-45% (w/w), 25-35% (w/w), 20-30% (w/w), or 30-50 (w/w) of the total carotenoids of the composition. Each possibility represents a separate embodiment of the invention.

In some embodiments, the composition comprises phytofluene in the amount of 1-10% (w/w), 3-12% (w/w), 4-14% (w/w), 5-10% (w/w), 8-15% (w/w), or 2-9% (w/w) of the total carotenoids of the composition. Each possibility represents a separate embodiment of the invention.

In some embodiments, the composition comprises zeta carotene in the amount of 4-20% (w/w), 6-18% (w/w), 5-15% (w/w), 6-12% (w/w), 9-17% (w/w), or 10-17% (w/w) of the total carotenoids of the composition. Each possibility represents a separate embodiment of the invention.

In some embodiments, the weight ratio of phytoene and phytofluene combined to zeta carotene ranges from 20:1 (w/w) to 3:1 (w/w), 15:1 (w/w) to 3:1 (w/w), 20:1 (w/w) to 6:1 (w/w), 15:1 (w/w) to 2:1 (w/w), 17:1 (w/w) to 4:1 (w/w), 16:1 (w/w) to 7:1 (w/w), 13:1 (w/w) to 8:1 (w/w), 10:1 (w/w) to 3:1 (w/w), or 15:1 (w/w) to 10:1 (w/w). Each possibility represents a separate embodiment of the invention.

In some embodiments, the composition further comprises an additional carotenoid. As used herein, "an additional carotenoid" refers to any carotenoid or a metabolite thereof, other than or being different from phytoene, phytofluene, and zeta carotene.

In some embodiments, the additional carotenoid is selected from lycopene, beta carotene, gamma carotene, and any combination thereof.

In some embodiments, the composition comprises lycopene in the amount of less than 10% (w/w), less than 7% (w/w), less than 5% (w/w), less than 3% (w/w), less than 2% (w/w), or less than 1% (w/w), of the total carotenoids in the composition, or any value and range therebetween. Each possibility represents a separate embodiment of the invention. In some embodiments, the composition comprises lycopene in the amount of 1-3% (w/w), 1-5% (w/w), 2-6% (w/w), 0.5-4.5% (w/w), 0.1-3% (w/w), 0.6-4.8% (w/w), or 2.5-4% (w/w) of the total carotenoids in the composition. Each possibility represents a separate embodiment of the invention.

In some embodiments, the composition comprises beta carotene in the amount of less than 10% (w/w), less than 7% (w/w), less than 5% (w/w), less than 3% (w/w), less than 2% (w/w), or less than 1% (w/w), of the total carotenoids in the composition, or any value and range therebetween. Each possibility represents a separate embodiment of the invention. In some embodiments, the composition comprises beta carotene in the amount of 1-3% (w/w), 1-5% (w/w), 2-6% (w/w), 0.5-4.5% (w/w), 0.1-3% (w/w), 0.6-4.8% (w/w), or 2.5-4% (w/w) of the total carotenoids in the composition. Each possibility represents a separate embodiment of the invention.

In some embodiments, the composition comprises gamma carotene in the amount of at least 0.15% (w/w), at least 0.18% (w/w), at least 0.2% (w/w), at least 0.25% (w/w), at least 0.35% (w/w), at least 0.5% (w/w), at least 0.75% (w/w), at least 0.9% (w/w), at least 1% (w/w), at least 1.2% (w/w), at least 1.35% (w/w), or at least 1.7% (w/w) of the total carotenoids in the composition, or any value and range therebetween. Each possibility represents a separate embodiment of the invention. In some embodiments, the composition comprises gamma carotene in the amount of 0.15-3% (w/w), 0.2-2% (w/w), 0.2-1.5% (w/w), 0.5-3% (w/w), 0.7-1.6% (w/w), 0.4-2.8% (w/w), or 1.2-3.2% (w/w) of the total carotenoids in the composition. Each possibility represents a separate embodiment of the invention.

In some embodiments, the composition comprises lycopene in the amount of less than 5% (w/w) of the total carotenoids in the composition, beta carotene in the amount of less than 5% (w/w) of the total carotenoids in the composition, gamma carotene in the amount of 0.2-1.5% (w/w) of the total carotenoids in the composition, or any combination thereof.

In some embodiments, the composition comprises an additional carotenoid in the amount of 0.1-3% (w/w), 0.2-3.5% (w/w), 0.5-2.5% (w/w), 0.15-1.75% (w/w), 0.35-2.75% (w/w), 0.8-4% (w/w), 1-5% (w/w), or 1.5-4.75% (w/w). Each possibility represents a separate embodiment of the invention.

In some embodiments, lycopene in the amount of less than 5% (w/w) of total carotenoids in the composition.

In some embodiments, the composition comprises a total carotenoids amount of 5-25% (w/w), 10-15% (w/w), 12-35% (w/w), 3-17% (w/w), 2-20% (w/w), or 1-30% (w/w) of the composition. Each possibility represents a separate embodiment of the invention.

In some embodiments, the composition further comprises a tocopherol (e.g., vitamin E). In some embodiments, the composition comprises a tocopherol in the amount of 1-30% (w/w), 3-35% (w/w), 5-25% (w/w), 2-20% (w/w), 4-41% (w/w), 8-32% (w/w), or 13-39% (w/w) of the composition. Each possibility represents a separate embodiment of the invention.

In some embodiments, the weight ratio of phytoene and phytofluene combined to a tocopherol ranges from 20:1 (w/w) to 3:1 (w/w), 15:1 (w/w) to 3:1 (w/w), 20:1 (w/w) to 6:1 (w/w), 17:1 (w/w) to 4:1 (w/w), 16:1 (w/w) to 7:1 (w/w), 13:1 (w/w) to 8:1 (w/w), 10:1 (w/w) to 3:1 (w/w), or 15:1 (w/w) to 10:1 (w/w). Each possibility represents a separate embodiment of the invention.

In some embodiments, the weight ratio of zeta carotene to a tocopherol ranges from 3:1 (w/w) to 1:3 (w/w), 3:1 (w/w) to 1:2 (w/w), 3:1 (w/w) to 1:1 (w/w), 2:1 (w/w) to 1:1 (w/w), 2:1 (w/w) to 1:2 (w/w), 2:1 (w/w) to 1:3 (w/w), 1:1 (w/w) to 1:2 (w/w), or 1:1 (w/w) to 1:3 (w/w). Each possibility represents a separate embodiment of the invention.

In some embodiments, the composition further comprises a phytosterol.

In some embodiments, the phytosterol is selected from: Cholesterol Brassicasterol, Campesterol, Stigmasterol, β-Sitosterol, Δ5-Avenasterol, Δ7-Avenasterol, Δ7-Stigmasterol, and any combination thereof.

In some embodiments, the composition comprises a phytosterol in the amount of 1-20% (w/w), 2-19% (w/w), 10-25% (w/w), 5-25% (w/w), 8-16% (w/w), 6-18% (w/w), 3-20% (w/w), 4-17% (w/w), or 5-15% (w/w), of the composition. Each possibility represents a separate embodiment of the invention. Each possibility represents a separate embodiment of the invention.

In some embodiments, the weight ratio of phytoene and phytofluene combined to a phytosterol ranges from 20:1 (w/w) to 3:1 (w/w), 15:1 (w/w) to 3:1 (w/w), 20:1 (w/w) to 6:1 (w/w), 17:1 (w/w) to 4:1 (w/w), 16:1 (w/w) to 7:1 (w/w), 13:1 (w/w) to 8:1 (w/w), 10:1 (w/w) to 3:1 (w/w), or 15:1 (w/w) to 10:1 (w/w). Each possibility represents a separate embodiment of the invention.

In some embodiments, the weight ratio of zeta carotene to a phytosterol ranges from 6:1 (w/w) to 2:1 (w/w), 5:1 (w/w) to 2:1 (w/w), 4:1 (w/w) to 2:1 (w/w), 3:1 (w/w) to 2:1 (w/w), 6:1 (w/w) to 3:1 (w/w), 5:1 (w/w) to 3:1 (w/w), 4:1 (w/w) to 3:2 (w/w), or 6:1 (w/w) to 3:1 (w/w). Each possibility represents a separate embodiment of the invention.

Methods for determining the amounts of phytonutrients, such as carotenoids, are common and would be apparent to one of ordinary skill in the art. Non-limiting examples for such methods include, but are not limited to, gas chromatography, liquid chromatography, and mass spectrometry.

In some embodiments, the composition is an oral composition or a topical composition. In some embodiments, the composition is a pharmaceutical or a nutraceutical composition. In some embodiments, the composition comprises a pharmaceutical or a nutraceutical acceptable carrier or excipient. In some embodiments, the composition is a cosmeceutical composition. In some embodiments, the composition comprises a cosmeceutical acceptable excipient. In some embodiments, a composition as described herein in an ingestible skin composition.

In some embodiments, an oral composition is in the form of a soft gel capsule. In some embodiments, an oral composition is in the form of a beverage, a shot, a gummy, or a powder. In some embodiments, an oral composition is mixed or assimilated into a food stuff, such as chocolate, ice cream, or others.

In some embodiments, the composition of the invention is for use in reducing collagen loss, increasing collagen levels, or both.

Methods for measuring collagen levels are common and would be apparent to one of ordinary skill in the art. Non-limiting examples of methods for determining or measuring collagen levels include, immunofluorescent microscopy, electron microscopy, western-blot, and qRT-PCR, some of which are exemplified hereinbelow. For example, by comparing collagen levels measured in the presence and absence of the composition of the invention, collagen loss, increased collagen levels, or both, can be determined.

In some embodiments, the composition of the invention has an activity of increasing collagen levels. In some embodiments, an activity of increasing collagen levels comprises any activity selected from: increasing collagen synthesis, increasing collagen weight per tissue weight, increasing collagen fibrils number, length, or both, increasing the amount of natively or properly folded collagen, reducing the amount of structurally damaged collagen, and any combination thereof.

In some embodiments, collagen comprises pro-collagen 3, collagen 3, collagen 1a1, or any combination thereof.

In some embodiments, collagen is pro-collagen 3. In some embodiments, collagen is collagen 3. In some embodiments, collagen is collagen 1a1. In some embodiments, collagen is a combination of collagen 3, pro-collagen 3, and collagen 1a1.

In one embodiment, the composition of the invention can be provided to the individual *per-se.* In one embodiment, the composition of the present invention can be provided to the individual as part of a pharmaceutical composition or a nutraceutical composition comprising a pharmaceutically acceptable carrier.

In one embodiment, a "pharmaceutical composition", a "cosmeceutical composition" or a "nutraceutical composition" refers to a preparation of a composition as described herein with other chemical components such as physiologically suitable carriers and excipients. The purpose of a pharmaceutical composition, cosmeceutical composition, or a nutraceutical composition is to facilitate administration of the composition to an organism.

In some embodiments, a process for producing a composition comprising phytoene in the amount of 20-30% (w/w), phytofluene in the amount of 5-10% (w/w) of, zeta carotene in the amount of 5-15% (w/w), and an acceptable carrier, is provided. In some embodiments, the process comprises extracting a golden tomato as disclosed herein. In some embodiments, a composition of the invention comprises a golden tomato extract produced by the herein disclosed process.

In one embodiment, "a combined preparation" defines especially a "kit of parts" in the sense that the combination partners as defined above can be dosed independently or by use of different fixed combinations with distinguished amounts of the combination partners i.e., simultaneously, concurrently, separately or sequentially. In some embodiments, the parts of the kit of parts can then, e.g., be administered simultaneously or chronologically staggered, that is at different time points and with equal or different time intervals for any part of the kit of parts. The ratio of the total amounts of the combination partners, in some embodiments, can be administered in the combined preparation. In one embodiment, the combined preparation can be varied, e.g., in order to cope with the needs of a patient subpopulation to be treated or the needs of the single patient which different needs can be due to a particular disease, severity of a disease, age, sex, or body weight as can be readily made by a person skilled in the art.

In one embodiment, the phrases "physiologically acceptable carrier" and "pharmaceutically acceptable carrier" which be interchangeably used refer to a carrier or a diluent that does not cause significant irritation to a mammal and does not abrogate the biological activity and properties of the administered composition. An adjuvant is included under these phrases.

In one embodiment, "excipient" refers to an inert substance added to a composition to further facilitate administration of an active ingredient. In one embodiment, excipients include calcium carbonate, calcium phosphate, various sugars and types of starch, cellulose derivatives, gelatin, vegetable oils and polyethylene glycols.

Techniques for formulation and administration of drugs are found in "Remington's Pharmaceutical Sciences," Mack Publishing Co., Easton, PA, latest edition, which is incorporated herein by reference in its entirety.

In one embodiment, suitable routes of administration, for example, include oral, rectal, transmucosal, transnasal, intestinal or parenteral delivery, including intramuscular, subcutaneous and intramedullary injections as well as intrathecal, direct intraventricular, intravenous, intraperitoneal, intranasal, or intraocular injections.

According to some embodiments, a method for treating or preventing collagen loss in a subject in need thereof, comprising administering to the subject a therapeutically effective amount of the composition of the invention, is provided.

In some embodiments, the subject is afflicted with a collagen-loss related disease.

In some embodiments, the subject is a healthy subject. In some embodiments, the subject is afflicted with collagen loss due to any one of: aging, exposure to an environmental factor such as UV, pollution, and smoke, lack of sleep, stress, or any combination thereof.

In some embodiments, a subject afflicted with a collagen-loss related disease is characterized by having: reduced skin elasticity, reduced epidermal thickness, increased skin vulnerability to damage (such as a mechanical damage), increased skin wrinkles, sagging, or both, and any combination thereof.

Methods for determining any one of skin elasticity or epidermal thickness are common and would be apparent to one of ordinary skill in the art, and non-limiting examples of such methods include, but are not limited to skin suction or indentation and subsequent skin displacement detection by optical measurement, and histological analysis of a skin biopsy.

According to some embodiments, a method for inhibiting or reducing any one of the amount, the activity, or both, of any one of: a MMP, MPO, superoxide (SO), elastase, nitric oxide (NO), and any combination thereof, in a subject in need thereof, comprising administering to the subject a therapeutically effective amount of the composition of the invention, is provided.

In one embodiment, MMP is MMP-9, MMP-8, MMP-1, or any combination thereof.

Methods for determining the amount, the activity, or both, of any one of: MMP, MPO, SO, elastase, NO, are common and would be apparent to one of skill in the art. Non-limiting example of these methods include, but are not limited to, ELISA, immunohistochemistry, oxidation assays, and enzymatic assays, such as exemplified hereinbelow, and/or others.

In some embodiments, inhibiting or reducing is by 50-60%, 50-75%, 50-90%, 50-99%, 65-95%, 70-90%, or 75-99%, compared to control. Each possibility represents a separate embodiment of the invention.

As used herein, a control comprises the skin of a healthy subject. In some embodiments, the control is a healthy skin sample derived, isolated, or obtained from the same subject (e.g., the subject afflicted with the collagen-loss related disease).

In some embodiments, administering comprises orally administering. In some embodiments, administering comprises topically administering. In some embodiments, administering comprises combined orally administering and topically administering.

In some embodiments, a collagen-loss related disease is selected from: an age-related disease, a skin disease, and an inflammatory disease.

As used herein, "age-related disease" refers to a disease or a condition associated therewith having an incidence which increases rapidly with age. As used herein, the term "rapidly" is exponentially.

Non-limiting examples of conditions associated with age, which can be collectively termed 'lesser ailments of aging' (LAA) include, but are not limited to general muscle weakness, low temperature intolerance, age related cognitive decline including also minor memory lapses, skin wrinkles, and slow healing of bruises in the skin, wasting (total weight loss) muscle volume loss and bone density decrease. Non-limiting examples of age-related diseases include cardiovascular disease, cancer, arthritis, dementia, cataract, osteoporosis, metabolic diseases including diabetes, increased cholesterol and deterioration in lipid profile, hypertension, and neurodegenerative diseases including but not limited to Alzheimer's disease.

As used herein, the phrase "skin disease" refers to any skin pathological condition or a symptom thereof. In one embodiment, collagen loss is a pathogenic factor leading to the development of a skin disease. In one embodiment, collagen loss is a pathophysiological factor resulting from the onset, initiation, progression, or any combination thereof, of a skin disease. In one embodiment, collagen loss is a condition characteristic of a developing or an ongoing skin disease.

In some embodiments, a skin disease is an immune-related skin disease. In one embodiment, an immune-related skin disease is an inflammatory skin disease, an autoinflammatory skin disease, or an autoimmune skin disease.

As used herein, the phrase "inflammatory disease" refers to any disease involving a multicomponent response of an organism (e.g., cells of the immune system, molecular signal mediators such as cytokines, etc.) to a harmful exogenous entity, for example, bacteria, fungi, viruses, protozoa, allergens, etc.

In some embodiments, skin disease is characterized or determined based on skin damage. In some embodiments, skin damage is induced by any one of: radiation, oxidative stress, DNA damage, telomere shortening, inflammation, tobacco usage, and any combination thereof.

In some embodiments, radiation comprises any radiation wavelength within the light spectrum. As used herein, the term "light spectrum" encompasses wave lengths ranging from 10⁻⁹ m to 10⁻³ m. In some embodiments, radiation wavelength within the light spectrum comprises UV radiation, visible light radiation, infrared radiation, or a combination thereof. In some embodiments, an exposure to radiation comprises an exposure to sunlight.

As used herein, the term "ultraviolet (UV)" encompasses any wavelength of the UV range. In some embodiments, UV is UV radiation. In some embodiments, UV radiation is UVA radiation, UVB radiation, UVC, or any combination thereof.

As used herein, the term "infrared" encompasses wavelength spanning from 700 nm to 1,000 nm (having frequency of 430 THz to 300 GHz).

In some embodiments, the subject has increased amount, increased activity, or both, of any one of: MMP, MPO, SO, elastase, NO, and any combination thereof, in the skin, systemically, or both, compared to control.

In some embodiments, increased is by 1-10%, 5-30%, 15-50%, 25-75%, 70-150%, 100-350%, 250-550%, 500-750%, or 700-1,000% compared to control. Each possibility represents a separate embodiment of the invention.

As used herein, the terms "treatment" or "treating" of a disease, disorder, or condition encompasses alleviation of at least one symptom thereof, a reduction in the severity thereof, or inhibition of the progression thereof. Treatment need not mean that the disease, disorder, or condition is totally cured. To be an effective treatment, a useful composition herein needs only to reduce the severity of a disease, disorder, or condition, reduce the severity of symptoms associated therewith, or provide improvement to a patient or subject's quality of life.

As used herein, the term "prevention" of a disease, disorder, or condition encompasses the delay, prevention, suppression, or inhibition of the onset of a disease, disorder, or condition. As used in accordance with the presently described subject matter, the term "prevention" relates to a process of prophylaxis in which a subject is exposed to the presently described compositions or formulations prior to the induction or onset of the disease/disorder process. This could be done where an individual has a genetic pedigree indicating a predisposition toward occurrence of the disease/disorder to be prevented. For example, this might be true of an individual whose ancestors show a predisposition toward certain types of, for example, inflammatory disorders. The term "suppression" is used to describe a condition wherein the disease/disorder process has already begun but obvious symptoms of the condition have yet to be realized. Thus, the cells of an individual may have the disease/disorder, but no outside signs of the disease/disorder have yet been clinically recognized. In either case, the term prophylaxis can be applied to encompass both prevention and suppression. Conversely, the term "treatment" refers to the clinical application of active agents to combat an already existing condition whose clinical presentation has already been realized in a patient.

In some embodiments, preventing comprises reducing the disease severity, delaying the disease onset, reducing the disease cumulative incidence, or any combination thereof.

As used herein, the terms "subject" or "individual" or "animal" or "patient" or "mammal," refers to any subject, particularly a mammalian subject, for whom therapy is desired, for example, a human.

In the discussion unless otherwise stated, adjectives such as "substantially" and "about" modifying a condition or relationship characteristic of a feature or features of an embodiment of the invention, are understood to mean that the condition or characteristic is defined to within tolerances that are acceptable for operation of the embodiment for an application for which it is intended. Unless otherwise indicated, the word "or" in the specification and claims is considered to be the inclusive "or" rather than the exclusive or, and indicates at least one of, or any combination of items it conjoins.

It should be understood that the terms "a" and "an" as used above and elsewhere herein refer to "one or more" of the enumerated components. It will be clear to one of ordinary skill in the art that the use of the singular includes the plural unless specifically stated otherwise. Therefore, the terms "a", "an" and "at least one" are used interchangeably in this application.

For purposes of better understanding the present teachings and in no way limiting the scope of the teachings, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques.

In the description and claims of the present application, each of the verbs, "comprise", "include" and "have" and conjugates thereof, are used to indicate that the object or objects of the verb are not necessarily a complete listing of components, elements or parts of the subject or subjects of the verb.

Other terms as used herein are meant to be defined by their well-known meanings in the art.

Unless specifically stated or obvious from context, as used herein, the term "or" is understood to be inclusive.

Throughout this specification and claims, the word "comprise" or variations such as "comprises" or "comprising," indicate the inclusion of any recited integer or group of integers but not the exclusion of any other integer or group of integers.

As used herein, the term "consists essentially of", or variations such as "consist essentially of" or "consisting essentially of" as used throughout the specification and claims, indicate the inclusion of any recited integer or group of integers, and the optional inclusion of any recited integer or group of integers that do not materially change the basic or novel properties of the specified method, structure or composition.

As used herein, the terms "comprises", "comprising", "containing", "having" and the like can mean "includes", "including", and the like; "consisting essentially of or "consists essentially" likewise has the meaning ascribed in U.S. patent law and the term is open-ended, allowing for the presence of more than that which is recited so long as basic or novel characteristics of that which is recited is not changed by the presence of more than that which is recited, but excludes prior art embodiments. In one embodiment, the terms "comprises", "comprising", "having" are/is interchangeable with "consisting".

### EXAMPLES

Generally, the nomenclature used herein, and the laboratory procedures utilized in the present invention include chemical, molecular, biochemical, and cell biology techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); "Cell Biology: A Laboratory Handbook", Volumes I-III Cellis, J. E., ed. (1994); The Organic Chemistry of Biological Pathways by John McMurry and Tadhg Begley (Roberts and Company, 2005); Organic Chemistry of Enzyme-Catalyzed Reactions by Richard Silverman (Academic Press, 2002); Organic Chemistry (6th Edition) by Leroy "Skip" G Wade; Organic Chemistry by T. W. Graham Solomons and, Craig Fryhle.

### Material and Methods

### GTE preparation

Dry ground golden tomato (*Lycopersicon esculentum Mill.* (Fam. Solanaceae) or *Solanum lycopersicum*) pulp was extracted under follow conditions: extraction type-supercritical CO₂ extraction, pressure 360 bar, and a temperature of 60 °C. The amount of the crude extract from the raw material was 2.7% (w/w).

A non-limiting example for the compounds identified within a supercritical-extracted golden tomato, including their relative amounts, are specified hereinbelow (Table 1). The total extract weight was 7.55 gr.

**Table 1. Compounds identified within a crude GTE obtained by a supercritical extraction**

| **Compound** | **Weight %** |
|---|---|
| Lycopene | 0.55% |
| Tocopherols | 11.27% |
| 15-cis-Phytoene | 24.06% |
| Phytofluene | 6.75% |
| beta-carotene | 0.61% |
| zeta-carotene | 7.48% |
| gamma-carotene | 0.16% |
| Phytosterols | 3.15% |
| Total carotenoids | 39.61% |
| Phytoene and Phytofluene (PP) | 30.81% |

### Content in LycoDerm, LycoMato

Standardized to 10% of total carotenoids GTE extract is called Lumenato. Comparison of the active ingredients between Lumenato, Lycoderm and Lyc-O-Mato 6% are presented in **Table 2.**

**Table 2. Comparison of phytonutrients content**

| **Active ingredients** | Concentration, % | | | |
|---|---|---|---|---|
| | Lumenato (GTE of the present invention) | Dry golden tomato pulp | Lycoderm | Lycomato 6% |
| Lycopene | 0.1 | 0.07 | 4.7 | 6 |
| 15-cis-Phytoene | 6.4 | 0.34 | 2.0 | 1.8 |
| Phytofluene | 1.4 | 0.19 | 1.0 | 0.8 |
| beta-carotene | 0.1 | 0.05 | 0.2 | 0.3 |
| Zeta-carotene | 2.0 | 0.3 | 0.01 | 0.1 |
| Gamma-carotene | 0.03 | 0.03 | 0.01 | 0.01 |
| Tocopherols | 1.8 | 0.18 | 1.75 | 1.8 |
| Phytosterols | 0.9 | 0.09 | 1.0 | 1.5 |
| Total Phyto. & Phytof. | 7.8 | 0.53 | 3.0 | 2.6 |
| Total Carotenoids | 10.03 | 0.98 | 7.92 | 9.01 |

### Preparation of human neutrophils

Blood was drawn from healthy volunteers. Neutrophils, at 95% purity were obtained by Ficoll-Hypaque centrifugation, dextran sedimentation, and hypotonic lysis of erythrocytes within 1 hour after blood was drawn. Cells were counted and their viability was determined by trypan blue exclusion.

### Cell culture

Normal human dermal fibroblasts (NHFD) adult donor (PROMOCELL, Heidelberg, Germany), were cultured in fibroblast growth medium-2 (PROMOCELL) supplemented with fibroblast growth medium-2 (final supplemental concertation in the medium: Fetal calf serum 0.002% (v/v), basic fibroblast recombinant human growth factor 1 mg/ml, and recombinant human insulin 5 µg/ml), 2 mM L-glutamine; 100 U/ml penicillin; 100 µg/ml streptomycin (Beit-Haemek, Israel). The cells were maintained at 37 °C in humidified atmosphere containing with 5% CO₂. NHDFs were seeded on 24-well plates when reached more than 80% confluency.

### Immunofluorescence analysis

For immunofluorescence detection, NHFD were fixed with methanol at 20 °C for 3 min followed by wash in phosphate buffer saline (PBS). Fixed NHFDs were incubated with anti-collagen-3 antibodies (Santa Cruz) 1:50 in 5% (w/v) BSA/PBS for 90 min at room temperature. The cells were washed three times in PBS and incubated with Cy3 anti-mouse, (1:100 in 5% (w/v) BSA/PBS; Jackson ImmunoResearch Laboratories, Inc, PA, USA) for 60 min at room temperature. The cells were washed three times in PBS and the nuclei were stained with DAPI. Then final wash was performed, and the cells were analyzed using fluorescence microscopy (Olympus, BX60, Hamburg, Germany). Fluorescence intensity was determined for Collagen-3 using CellProfiler Program. The % of fluorescence area was determined using CellProfiler program.

### Superoxide generation

Cytochrome C reduction - The production of the superoxide anion by neutrophils was measured as the superoxide dismutase inhibitable reduction of ferricytochrome c by the microtiter plate technique. Cells (2.5 × 10⁵/well) were suspended in 100 µl Hanks' Balanced Salts Solution (HBSS) containing ferricytochrome c (150 mM). The reduction of acetyl ferricytochrome c was followed by the change of absorbance at 550 nm at 2-minute intervals on a Thermomax Microplate Reader (Molecular Devices, Melno Park, CA). The maximal rates of superoxide generation were determined and expressed as nanomoles O₂⁻/10⁶ cells/min using the extinction coefficient E₅₅₀ =21 mM⁻¹·cm⁻¹.

Amplex red - Horse Radish Peroxidase (HRP)-dependent oxidation of the highly sensitive fluorescent biosensor Amplex red was utilized. The oxidation of Amplex red occurs outside the cells by HRP, which traps H₂O₂ as soon as it is generated by spontaneous dismutation of O₂⁻, the first product of NADPH oxidase. Resting and activated neutrophils (2 × 10⁴/well) were suspended in KRPG buffer (phosphate buffer, 145 mM NaCl, 4.86 mM KCl, 1.22 mM MgSO₄, 5.5 mM D-glucose, 0.54 mM CaCl₂, pH 7.35) containing HRP (0.1 unit/ml) and Amplex red (50 µM). Fluorescence was recorded in a microplate reader with 535 nm excitation and 595 nm emission wavelengths. Background fluorescence was measured in the absence of microglial cells.

### Myeloperoxidase (MPO) activity

100 µl of 37 °C O-dianisidine hydrochloride solution (1 mg O-dianisidine, 10 ml phosphate buffer pH 6.0 + 0.0015% H₂O₂) were added to 100 µl supernatant in a 96-well plate immediately before the optical density was followed by the change of absorbance at 450 nm at 2 minute intervals on a Thermomax Microplate Reader (Molecular Devices, Melno Park, CA).

Tocopherol and phytosterol were added to 5 × 10⁵ human neutrophils 20 min before addition of 100 ng/ml TNFα and maintained overnight at 37 °C in a humidified atmosphere containing 5% CO₂. MPO activity was measured in the supernatant of the cultured neutrophils.

Zeta carotene, phytoene, tocopherol, phytosterol, or combinations thereof, were added to 5 × 10⁵ human neutrophils 20 min before addition of 100 ng/ml TNFα and maintained overnight at 37 °C in humidified atmosphere containing 5% CO₂. MPO activity was measured as escribed above.

### MMP-9

Human MMP-9 concentrations in cell culture supernatants were quantified by ELISA kits (R&D systems Minneapolis, MN, USA).

### MMP-8 (collagenase)

Human MMP-8 concentrations in cell culture supernatants were quantified by ELISA kits (OriGene, Technologies, Inc. Rockville, MD, USA).

### Elastase activity

N-methoxysuccinyl-ala-ala-pro-Val-P-nitroanilide was added to 100 µl of supernatant to reach a final concentration of 0.5 mM, and thereafter incubated overnight at 37 °C. The absorbance at 405 nm was measured on a Thermomax Microplate Reader (Molecular Devices, Melno Park, CA).

### Isolation and culture of macrophages

Peritoneal macrophages were collected from the peritoneal cavity of 6-8 weeks old male ICR mice (Harlan, Israel) after an intraperitoneal injection of 1.5 ml of thioglycollate broth (4%) 4 days before harvest. Peritoneal macrophages were washed three times with PBS followed by hypotonic lysis of erythrocytes, yielding a highly enriched (90-95%) macrophage cell population. Macrophages were identified by FACS analysis using FITC-conjugated rat anti-mouse F4/80 (MCA497F) (Serotec, Oxford , England) by flow microfluorimetry on FACS (Becton Dickinson, Mountain View, CA). For each sample, 10,000 light scatter-gated viable cells were analyzed. Peritoneal macrophages (1 × 10⁶ cells/well) were cultured in 96-well plates at 37 °C in 5% CO₂ atmosphere in RPMI 1640 medium containing 10% FCS, 2 mM L-glutamine; 100 U/ml penicillin; 100 µg/ml streptomycin (Beit-Haemek, Israel). Cells were stimulated with 100 ng/ml LPS from *Salmonella enterica* serotype typhimurium in the absence or presence of the ingredients. LPS was purchased from Sigma, Israel.

All compounds were dissolved in DMSO, the volume of DMSO did not exceed 0.1-0.2% in the test plates. To the control plates appropriate volumes of DMSO (0.1-0.2%) were added. The % inhibition in each tube test was calculated in relation to its control.

### Nitric oxide (NO) production assay

NO levels in the supernatant of cell cultures were determined by assaying nitrite concentration using Griess reagent and sodium nitrite as a standard.

### Cell survival

Cell viability was assessed by cell count using trypan blue exclusion or by the colorimetric MTT ([3-4, 5-dimethylthiazol-2-yl]-2,5-diphenyl-tetrazolium) metabolic activity assay. For MTT measurement cells were cultured in 96 well plates. MTT was dissolved in medium (5 mg/ml) and added to each sample in an amount equal to 10% of the culture medium volume. After incubation for 4 h, the formazan crystals were dissolved in 100 mM HCl, 10% Triton X-100 all in isopropanol in equal volume to culture medium, the medium only served as a background. Absorbance intensity measured by a Thermomax Microplate Reader (Molecular Devices, Melno Park, CA, USA) at 570 nm with a reference wavelength of 690 nm.

### Pro-collagen-3

Human pro-collagen-3 concentrations in cell-culture supernatants were quantified by a Human PIIINP (N-terminal pro-collagen III propeptide) ELISA kit (Elabscience Biotechnology Inc. Houston, Texas, USA). Since the concentration of pro-collagen-3 in the supernatants of the fibroblasts was very low, the supernatants were concentrated by evaporation.

### Collagen-3

Human collagen-3 concentrations in cell-culture lysates and supernatants were quantified by a human collagen, type III, alpha 1 (COL3A1) ELISA kit (Cusabio Technology Inc., Houston, Texas, USA).

### Collagen 1a1

Cells were incubated with Lumenato for 24 h and then hydrogen peroxide (H₂O₂) was added, and cells were incubated for additional 24 h to induce oxidative stress. The incubation medium was collected and the levels of collagen1a1 protein secretion to the medium were analyzed using a specific ELISA detection kit.

### Statistical analysis

Significant differences between evaluated parameters were determined by ANOVA using GraphPad Prism 5 (GraphPad Software Inc., San Diego, CA, USA) followed by multiple comparisons Bonferroni post hoc correction. *P* value of less than 0.05 were considered statistically significant. Significance: * *- p<* 0.05 , ** *-p*<0.001, *** *-p*<0.0001.

### EXAMPLE 1

### GTE reduces secretion of degrading enzymes from stimulated neutrophils

In order to study the effect of GTE on the release of enzymes that have a potential to cause damage to collagen, the inventors first determined the kinetics of the release of such enzymes from activated neutrophils. Neutrophils were activated by 100 ng/ml TNFα or 100 ng/ml IL8 that are released from the skin cells during exposure to UV and activated. In addition, neutrophils were activated with 5 × 10⁻⁷ fMLP for comparison. As shown in **Fig. 1****,** addition of TNFα to human neutrophils caused a significant (p<0.001) release of MMP-9 at 2 hr of activation (16.52 ± 0.5 ng/ml) that persisted at the same level at 4 hr. Addition of IL8 caused a release of MMP-9 in a dose dependent manner during the 4 hr of activation. A significant (p<0.001) release of MMP-9 was detected at 2 hr of activation with IL8 (9.12 ± 0.2 ng/ml) which was further significantly (p<0.0001) increased at 4 hr to 17.86 ± 0.4 ng/ml. fMLP induced a rapid and significant (p<0.001) release of MMP-9 at 30 min of activation (10.41+0.4 ng/ml) that was gradually increased during the 4 hr of activation to 13.45+0.3 ng/ml. As shown in **Fig. 2****,** the addition of either TNFα or IL8 induced a significant (p<0.0001) release of MPO at 2 hr of activation, that was further increased at 4 h to 0.28 ± 0.001 ng/ml and 0.4 ± 0.08 ng/ml respectively. fMLP induced a rapid and significant (p<0.0001) release of MPO at 30 min to 0.54 ± 0.004 ng/ml that persisted at this level at 4 hr. Activation of neutrophils for 24 hr did not augment the release of MMP-9 or MPO compared with 4 hr (data not shown).

Neutrophils were activated by TNFα or IL8 for 4 hr and by fMLP for 30 min, in order to study the effect of GTE on the release of MMP-9 or MPO. GTE was added to the neutrophils for 10 min at 37 °C before activation. As shown in **Fig. 3****,** the addition of GTE in a concentration range of 26-210 µg/ml inhibited the release of MMP-9 from neutrophils stimulated by either TNFα, IL8, or fMLP, in a dose dependent manner. Maximal inhibition of MMP-9 secretion was 95.4 ± 4.6%, 83.19 ± 12.0% or 76.6 ± 12.0% from neutrophils stimulated by either TNFα, IL8 or fMLP, respectively. Similarly, GTE induced a dose dependent inhibition of MPO with maximal inhibition of 46.0 ± 6.7%, 33.86 ± 0.7% or 38.5 ± 4.5% from neutrophils stimulated by TNFα, IL8 or fMLP, respectively **(****Fig. 4****).**

Next, the effect of GTE on superoxide release from neutrophils was analyzed by cytochrome C reduction. The analysis was performed immediately after addition of each of the aforementioned agonists (e.g., TNFα, IL8, etc.) since superoxide is unstable. As shown in **Fig. 5****,** the addition of GTE for 10 min prior to stimulation induced a dose dependent inhibition of superoxide production, reaching maximal inhibition of 63.27 ± 13%, 67.55 ± 3.4%, and 56.81 ± 2.4%, neutrophils were stimulated by TNFα, IL8, and fMLP, respectively.

### EXAMPLE 2

### GTE reduces collagen loss in fibroblast-neutrophils cocultures

In order to study the effect of GTE on the damage of collagen-3 induced by activated neutrophils the inventors used optimal conditions of cultured fibroblasts and of neutrophils. 1 × 10⁵ fibroblasts were plated for 24 hr to obtain confluent cultures. Addition of 2 × 10⁵ activated neutrophils induced a significant collagen-3 damage of about 30% but did not ruin the cultures, as shown by immune-fluoresce staining of collagen-3 **(****Figs. 6A-****6N).** Cell viability was measured in each treatment and was found to be un-affected by the addition of stimulated neuropils in the absence or presence of GTE. The concentration of neutrophils used in co-culture experiments was lower than used to study neutrophils **(****Figs. 1-5****).** Neutrophils were incubated with GTE for 10 min at 37 °C before being added to the cultures for 24 hr. As shown by immune-fluoresce staining of collagen 3 **(****Figs. 6A-6N****),** the addition of neutrophils pre-incubated with GTE in the range of 6.5-104 µg/ml before their addition to the fibroblasts cultures resulted with a dose dependent inhibition of collagen 3 damage. The maximal prevention of collagen damage was reached at 52 µg/ml GTE (around 80% protection). Quantification of collagen-3 levels in the cultures, which was determined by densitometry and presented in **Fig. 6O****,** were greater than other tomato-derived compositions **(****Figs. 6P-6Q****).** The presence of Lumenato in the co-cultures caused a dose-dependent increase in the secreted pro-collagen-3 to 68.21±7.1 pg/ml in the presence of 104 ug/ml Lumenato **(****Fig. 6S****)** subtracting the level of pro-collagen-3 induced by the neutrophils (marked with a dash horizontal line) indicated an increase of around 10 pg/ml in the presence of 104 ug/ml Lumenato. Further, the addition of Lumenato to fibroblasts overnight did not affect fibroblast cell number as measured by MTT or by DAPI staining (data not shown). Nonetheless, the addition of Lumenato induced a significant increase of pro-collagen-3 levels in a dose-dependent manner in the supernatants **(****Fig. 18****).**

Treatment of NHDF cells with 50 µM H₂O₂ resulted in about 10-fold reduction in collagen 1a1 secretion **(****Fig. 19****).** Lumenato already at the lowest concentration used, completely abolished the reduction in collagen secretion. This result, in view of Lumenato's ability to also reduce MMP1 secretion suggest Lumenato is a promising agent for increased skin collagen levels and reduce skin ageing, *in vivo.*

The amounts/levels of released degrading enzymes under the aforementioned coculture system, (as described in **Fig. 6****)** were measured in the supernatant after 24 hr of culture. The concentrations of GTE used in the coculture experiments was lower than that used to study their effect on neutrophils **(****Figs. 1-5****)** since in the coculture experiments lower concentration of neutrophils was used (2.5 × 10⁵/ml compared with 5 × 10⁶/ml). As shown in **Fig. 7A****,** a dose dependent inhibition of MMP-9 was observed for GTE, but not in other tomato-derived compositions **(****Figs. 7B-7C****).** The inhibition of GTE was about 12.5 ± 3.9% inhibition. MPO release, which was determined based on its activity in the supernatant, was significantly inhibited at GTE concentration as low as 13 µg/ml, which gradually increased, reaching a 45.8 ± 5.6% inhibition at maximal concentration tested **(****Fig. 8A****).** GTE MPO-inhibiting effect was about 2-fold compared to other tomato-derived compositions **(****Figs. 8B-****8C).** The effect of GTE on superoxide production was studied by Amplex red, which was performed immediately after stimulation of the neutrophils (superoxides are unstable). As shown in **Fig. 9****,** GTE induced a significant dose dependent of inhibition reaching 78.5 ± 0.9% inhibition. The beneficial effect of GTE in the coculture was further exemplified by inhibition of MMP-8 (collagenase), as presented in **Fig. 10****.** GTE induced a gradual inhibition of MMP-8 release, with a maximal inhibition of 43.0 ± 7.1% achieved at 26 µg/ml of GTE, which persisted under greater GTE concentrations. Elastase activity was not significantly elevated in the presence of GTE in cocultures of stimulated neutrophils and fibroblasts **(****Fig. 11****).**

### EXAMPLE 3

### GTE reduces nitric oxide production in stimulated macrophages

Macrophages were stimulated with LPS and thereafter incubated with increased concentrations of either filtered pulp or GTE. As shown in **Fig. 12****,** GTE was 2-fold more effective in inhibiting nitric oxide (NO) production compared to the filtered pulp. GTE was found to inhibit NO production in a dose dependent manner starting at a concentration as little as 5 µg/ml, showing approximately 20% inhibition. The level of NO production inhibition continuously increased to approximately 55% in the presence of GTE concentration of 100 µg/ml.

### EXAMPLE 4

### Compositions of multiple phytonutrients synergistically inhibit MPO activity

The inventors examined the effect of multiple phytonutrients on MPO activity in in stimulated neutrophils. The sole addition of tocopherol or phytosterol had no effect on the release and activity of MPO from neutrophils activated with TNFα **(****Figs. 13A-13B****).** However, supplementation of 225 µg/ml of a tocopherol and 62.5 µg/ml of a phytosterol resulted in a synergistic effect, with approximately 20% inhibition of MPO activity compared to the controls **(****Fig. 13C****).**

The inventors also examined the effect of adding zeta carotene with either phytoene, a tocopherol, or a phytosterol, on MPO activity. The sole addition of zeta carotene, a tocopherol, phytoene, or a phytosterol had no inhibitory effect to 5% inhibition of MPO activity, at most **(****Fig. 14****).** However, the addition of zeta carotene with any one of: a tocopherol, phytoene, or a phytosterol in the ratio present in GTE induced a synergistic inhibition of MPO activity or release from TNFα-activated neutrophils **(****Fig. 14****).**

### EXAMPLE 5

### Lumenato capsules having increased carotenoid bioavailability

The inventors have examined the bioavailability of carotenoids delivered within Lumenato capsules. The following carotenoids were quantified in the plasma at 1 week, 2 weeks, 3 weeks, and 4 weeks post administration. Phytoene, phytofluene and zeta-carotene which are the main carotenoids in Lumenato (the golden tomato extract) were absorbed well **(****Fig. 15****).** Zeta carotene was found to be absorbed extremely well, as reflected by a steep increase in plasma concentration **(****Fig. 15A****).**

### EXAMPLE 6

### Balancing oxidative stress induced damage in skin cells

The inventors then examined whether Lumenato, the golden tomato extract, enhances skin resilience and prevents oxidative stress induced-skin damage in human keratinocytes. Reactive oxygen species (ROS) such as H₂O₂ are known to induce inflammatory processes in the skin, increase the production of matrix metalloproteinase (MMPs) in skin cells, leading to collagen degradation.

The inventors used hydrogen peroxide, H₂O₂, to induce oxidative stress in KERTr human keratinocytes, and analyzed the effects on cell number (cell death) and the secretion of MMP1. Cells viability was determined using the XTT cell proliferation kit, and MMP1 levels were measured by ELISA. Treatment of the cells with H₂O₂ led to a dose dependent cell death **(****Fig. 16A****)** and increased secretion of MMP1 **(****Fig. 16B****).** Addition of Lumenato at the indicated concentrations before adding H₂O₂ increased cell viability and reduced the secretion of MMP1.

One possible explanation for the above-mentioned protective effect is the activation of the antioxidant response element transcription system (ARE/Nrf2). Thus, the inventors measured the activation of this transcriptional system using a reporter gene assay. A dose dependent activation of ARE/Nrf2 transcriptional activity by Lumenato was observed **(****Fig. 17****).**

### EXAMPLE 7

### Skin condition and appearance

The inventors assembled a baseline skin questionnaire which included 14 questions asking the participants about their skin condition and appearance before and after taking the study product, Lumenato, (e.g., at baseline and after 12 weeks). The results show **(Table 3)** significant improvement in levels of satisfaction in 11 out of 14 questions, (excluding questions numbers 3, 10, and 11).

**Table 3. Results of a baseline skin questionnaire**

| Question | Baseline | | | Week 12 | | | Wilcoxon signed rank test p-value |
|---|---|---|---|---|---|---|---|
| | Mean | Median | SD | Mean | Median | SD | |
| 1. My skin lacks elasticity | 3.17 | 3.0 | 0.85 | 2.43 | 2.0 | 0.98 | <0.001 |
| 2. My skin lacks firmness | 3.33 | 3.0 | 0.73 | 2.45 | 2.0 | 0.91 | <0.001 |
| 3. I have pigmentation spots on my hands | 2.53 | 2.0 | 1.23 | 2.57 | 2.5 | 1.16 | 0.8048 |
| 4. My skin acks hydration/feels dry | 3.57 | 4.0 | 1.00 | 2.83 | 3.0 | 1.15 | <0.001 |
| 5. My skin has a rough texture/lacks smoothness | 3.20 | 3.0 | 1.01 | 2.65 | 2.0 | 1.09 | <0.001 |
| 6. My skin has an uneven tone | 3.77 | 4.0 | 0.91 | 2.97 | 3.0 | 1.15 | <0.001 |
| 7. My skin appears dull/lacks brightness | 3.90 | 4.0 | 0.66 | 2.77 | 3.0 | 1.03 | <0.001 |
| 8. I have dark circles under my eyes | 3.72 | 4.0 | 1.11 | 3.02 | 3.0 | 1.11 | <0.001 |
| 9. I have fine lines/wrinkles | 3.87 | 4.0 | 0.72 | 3.35 | 4.0 | 1.02 | <0.001 |
| 10. I have deep wrinkles | 2.20 | 2.0 | 0.86 | 2.28 | 2.0 | 0.87 | 0.5349 |
| 11. I have a tendency to develop redness | 3.23 | 3.5 | 1.23 | 3.12 | 3.0 | 1.06 | 0.2929 |
| 12. I have dark spots (pigmentation spots) on my face | 3.52 | 4.0 | 1.17 | 3.12 | 3.0 | 1.11 | 0.0035 |
| 13. My skin looks and feels aged/unhealthy | 3.30 | 3.0 | 0.87 | 2.53 | 2.0 | 1.02 | <0.001 |
| 14. I am dissatisfied with the overall appearance of my skin | 3.88 | 4.0 | 0.85 | 2.80 | 3.0 | 1.04 | <0.001 |

Further, the inventors assembled a skin update questionnaire which included 14 questions which asking the participants about their skin condition and appearance while they were taking the study product, Lumenato (Weeks 4, 8, and 12). The results show **(Table 4)** a significant improvement in levels of satisfaction in 10 out of 14 questions (excluding questions number 3, 8, 11, and 12).

**Table 4. Results of skin update questionnaire**

| Question | Week 4 | | | Week 8 | | | Week 12 | | | Friedm an test p-value |
|---|---|---|---|---|---|---|---|---|---|---|
| | Me an | Medi an | SD | Me an | Medi an | SD | Me an | Medi an | SD | |
| 1. The test product has improved my skin's elasticity | 3.10 | 3 | 0.7 1 | 3.42 | 3 | 0.7 4 | 3.50 | 4 | 0.8 3 | <0.001 |
| 2. The test product has improved my skins firmness | 3.13 | 3 | 0.7 5 | 3.45 | 3 | 0.8 1 | 3.43 | 3 | 0.8 3 | 0.003 |
| 3. The test product has improved the pigmentation spots on my hands | 2.92 | 3 | 0.7 7 | 3.07 | 3 | 0.7 3 | 3.07 | 3 | 0.8 6 | 0.3679 |
| 4. The test product has improved my skin dryness/my skin feels more hydrated | 3.10 | 3 | 1.0 5 | 3.27 | 3 | 0.9 0 | 3.50 | 4 | 0.9 5 | 0.0042 |
| 5. The test product has improved my skin's texture/my skin feels smoother | 3.25 | 3 | 0.8 9 | 3.50 | 4 | 0.8 3 | 3.43 | 4 | 0.9 8 | 0.0464 |
| 6. The test product has improved the tone of my skin/my skin tone appears more even | 3.13 | 3 | 0.8 1 | 3.57 | 4 | 0.8 1 | 3.43 | 4 | 0.9 6 | <0.001 |
| 7. After using the test product, my skin appears brighter | 3.17 | 3 | 0.8 1 | 3.38 | 3 | 0.8 5 | 3.55 | 4 | 0.8 5 | <0.001 |
| 8. After using the test product, the dark circles around my eyes appear lighter | 2.97 | 3 | 0.8 6 | 3.05 | 3 | 0.9 3 | 3.05 | 3 | 0.8 9 | 0.0564 1 |
| 9. The test product has improved the appearance of fine lines/wrinkles | 3.02 | 3 | 0.7 9 | 3.20 | 3 | 0.8 4 | 3.38 | 3 | 0.8 8 | <0.001 |
| 10. The test product has improved the appearance of deep wrinkles | 2.93 | 3 | 0.7 6 | 3.10 | 3 | 0.8 6 | 3.20 | 3 | 0.7 5 | 0.0134 |
| 11. The test product has reduced skin redness | 3.22 | 3 | 0.8 8 | 3.25 | 3 | 0.9 1 | 3.37 | 3 | 0.7 6 | 0.4895 |
| 12. The test product has reduced skin appearance of my dark spots (pigmentation spots) | 3.08 | 3 | 0.8 1 | 3.20 | 3 | 0.8 2 | 3.27 | 3 | 0.9 0 | 0.2559 |
| 13. After using the test product, my skin looks and feels vounger/heathier | 3.22 | 3 | 0.8 0 | 3.52 | 4 | 0.8 5 | 3.58 | 4 | 0.7 9 | <0.001 |
| 14. The test product has improved the overall appearance of my skin | 3.23 | 3 | 0.8 5 | 3.67 | 4 | 0.8 2 | 3.58 | 4 | 0.8 9 | <0.001 |

While the present invention has been particularly described, persons skilled in the art will appreciate that many variations and modifications can be made. Therefore, the invention is not to be construed as restricted to the particularly described embodiments, and the scope and concept of the invention will be more readily understood by reference to the claims, which follow.

## Claims

1. A composition comprising: phytoene in the amount of 55-65% (w/w) of the total carotenoids in said composition, phytofluene in the amount of 10-20% (w/w) of the total carotenoids in said composition, zeta carotene in the amount of 15-25% (w/w) of the total carotenoids in said composition, tocopherol in the amount of 10-30% (w/w) of the total carotenoids in said composition, and an acceptable carrier.

2. The composition of claim 1, wherein the weight ratio of said phytoene and said phytofluene combined to said zeta carotene ranges from 15:1 (w/w) to 2:1 (w/w).

3. The composition of claims 1 or 2, further comprising an additional carotenoid selected from the group consisting of: lycopene, beta carotene, gamma carotene, and any combination thereof.

4. The composition of claim 3, comprising said lycopene in the amount of less than 5% (w/w) of the total carotenoids in said composition, said beta carotene in the amount of less than 5% (w/w) of the total carotenoids in said composition, said gamma carotene in the amount of 0.2-1.5% (w/w) of the total carotenoids in said composition, or a combination thereof.

5. The composition of any one of claims 1 to 4, comprising total carotenoids amount of 10-15% (w/w) of said composition.

6. The composition of any one of claims 1 to 5, further comprising a phytosterol.

7. The composition of claim 6, comprising said phytosterol in the amount of 5-15% (w/w) total carotenoids in said composition.

8. The composition of any one of claims 1 to 7, being a cosmeceutical composition.

9. The composition of any one of claims 1 to 8, for use in preventing or treating a skin disease **characterized by** collagen loss in a subject in need thereof.

10. The composition for use of claim 9, wherein said subject has increased amount, activity, or both, of any one of: a matrix metalloprotease (MMP), MPO, superoxide (SO), elastase, nitric oxide (NO), and any combination thereof, in the skin, compared to a skin of a healthy control subject.

11. The composition for use of claim 10, wherein said MMP is selected from: MMP-9, MMP-8, MMP-1, or any combination thereof.

12. The composition for use of claim 9, wherein said skin disease **characterized by** collagen loss is selected from the group consisting of: an age-related disease, and an inflammatory disease, and optionally wherein said skin disease comprises skin damage induced by any one of: radiation, oxidative stress, DNA damage, telomere shortening, inflammation, tobacco usage, and any combination thereof.

13. The composition for use of claim 12, wherein said radiation comprises UV radiation, visible light radiation, infrared radiation, or any combination thereof, and optionally wherein said UV radiation is UVA, UVB, UVC, or any combination thereof.

## Patentansprüche

1. Zusammensetzung, umfassend:
Phytoen in der Menge von 55-65 % (w/w) der gesamten Carotinoide in der Zusammensetzung,
Phytofluen in der Menge von 10-20 % (w/w) der gesamten Carotinoide in der Zusammensetzung,
Zeta-Carotin in der Menge von 15-25 % (w/w) der gesamten Carotinoide in der Zusammensetzung,
Tocopherol in der Menge von 10-30 % (w/w) der gesamten Carotinoide in der Zusammensetzung, und
einen akzeptablen Träger.

2. Zusammensetzung nach Anspruch 1, wobei das Gewichtsverhältnis des Phytoens und des Phytofluens kombiniert zu dem Zeta-Carotin von 15:1 (w/w) bis 2:1 (w/w) reicht.

3. Zusammensetzung nach Anspruch 1 oder 2, ferner umfassend ein weiteres Carotinoid ausgewählt aus der Gruppe bestehend aus: Lycopin, Beta-Carotin, Gamma-Carotin und jeglicher Kombination davon.

4. Zusammensetzung nach Anspruch 3, umfassend
das Lycopin in der Menge von weniger als 5 % (w/w) der gesamten Carotinoide in der Zusammensetzung,
das Beta-Carotin in der Menge von weniger als 5 % (w/w) der gesamten Carotinoide in der Zusammensetzung,
das Gamma-Carotin in der Menge von 0,2-1,5 % (w/w) der gesamten Carotinoide in der Zusammensetzung, oder eine Kombination davon.

5. Zusammensetzung nach einem der Ansprüche 1 bis 4, umfassend eine Gesamtcarotinoidmenge von 10-15 % (w/w) der Zusammensetzung.

6. Zusammensetzung nach einem der Ansprüche 1 bis 5, ferner umfassend ein Phytosterol.

7. Zusammensetzung nach Anspruch 6, umfassend das Phytosterol in der Menge von 5-15 % (w/w) der gesamten Carotinoide in der Zusammensetzung.

8. Zusammensetzung nach einem der Ansprüche 1 bis 7, welche eine kosmetische Zusammensetzung ist.

9. Zusammensetzung nach einem der Ansprüche 1 bis 8 zur Verwendung zum Vorbeugen oder Behandeln einer Hauterkrankung, welche durch Kollagenverlust gekennzeichnet ist, bei einem Individuum, das dessen bedarf.

10. Zusammensetzung zur Verwendung nach Anspruch 9, wobei das Individuum in der Haut verglichen mit einer Haut eines gesunden Kontrollindividuums eine erhöhte Menge, Aktivität oder beides aufweist von jeglichem aus: einer Matrix-Metalloprotease (MMP), MPO, Superoxid (SO), Elastase, Stickstoffmonoxid (NO) und jeglicher Kombination davon.

11. Zusammensetzung zur Verwendung nach Anspruch 10, wobei die MMP ausgewählt ist aus: MMP-9, MMP-8, MMP-1 oder jeglicher Kombination davon.

12. Zusammensetzung zur Verwendung nach Anspruch 9, wobei die durch Kollagenverlust gekennzeichnete Hauterkrankung ausgewählt ist aus der Gruppe bestehend aus: einer altersbedingten Erkrankung und einer entzündlichen Erkrankung, und gegebenenfalls wobei die Hauterkrankung Hautschädigung umfasst, induziert durch jegliches aus: Strahlung, oxidativem Stress, DNA-Schädigung, Telomerverkürzung, Entzündung, Tabaknutzung und jeglicher Kombination davon.

13. Zusammensetzung zur Verwendung nach Anspruch 12, wobei die Strahlung UV-Strahlung, sichtbare Lichtstrahlung, Infrarotstrahlung oder jegliche Kombination davon umfasst, und gegebenenfalls wobei die UV-Strahlung UVA, UVB, UVC oder jegliche Kombination davon ist.

## Revendications

1. Composition comprenant : du phytoène en une quantité de 55 à 65 % (p/p) des caroténoïdes totaux dans ladite composition, du phytofluène en une quantité de 10 à 20 % (p/p) des caroténoïdes totaux dans ladite composition, du zêta-carotène en une quantité de 15 à 25 % (p/p) des caroténoïdes totaux dans ladite composition, du tocophérol en une quantité de 10 à 30 % (p/p) des caroténoïdes totaux dans ladite composition, et un support acceptable.

2. Composition selon la revendication 1, dans laquelle le rapport pondéral dudit phytoène et dudit phytofluène combinés audit carotène zêta est compris entre 15:1 (p/p) et 2:1 (p/p).

3. Composition selon la revendication 1 ou 2, comprenant en outre un caroténoïde supplémentaire choisi dans le groupe comprenant : lycopène, bêta-carotène, gamma-carotène et toute combinaison de ceux-ci.

4. Composition selon la revendication 3, comprenant ledit lycopène en une quantité inférieure à 5 % (p/p) des caroténoïdes totaux dans ladite composition, ledit bêta-carotène en une quantité inférieure à 5 % (p/p) des caroténoïdes totaux dans ladite composition, ledit gamma-carotène en une quantité de 0,2 à 1,5 % (p/p) des caroténoïdes totaux dans ladite composition, ou une combinaison de ceux-ci.

5. Composition selon l'une quelconque des revendications 1 à 4, comprenant une quantité de caroténoïdes totaux de 10 à 15% (p/p) de ladite composition.

6. Composition selon l'une quelconque des revendications 1 à 5, comprenant en outre un phytostérol.

7. Composition selon la revendication 6, comprenant ledit phytostérol en une quantité de 5 à 15 % (p/p) de caroténoïdes totaux dans ladite composition.

8. Composition selon l'une quelconque des revendications 1 à 7, étant une composition cosmétique.

9. Composition selon l'une quelconque des revendications 1 à 8, pour utilisation dans la prévention ou le traitement d'une maladie de la peau **caractérisée par** une perte de collagène chez un sujet qui en a besoin.

10. Composition pour utilisation selon la revendication 9, dans laquelle ledit sujet présente une quantité, une activité, ou les deux, accrues d'un quelconque parmi : une métalloprotéase matricielle (MMP), une MPO, un superoxyde (SO), une élastase, un oxyde nitrique (NO), et toute combinaison de ceux-ci, dans la peau, par comparaison à une peau d'un sujet témoin en bonne santé.

11. Composition pour utilisation selon la revendication 10, dans laquelle ladite MMP est choisie parmi : MMP-9, MMP-8, MMP-1, ou toute combinaison de celles-ci.

12. Composition pour utilisation selon la revendication 9, dans laquelle ladite maladie de la peau **caractérisée par** une perte de collagène est sélectionnée dans le groupe comprenant : une maladie liée à l'âge et une maladie inflammatoire, et facultativement dans laquelle ladite maladie de la peau comprend des dommages cutanés induits par un quelconque parmi : un rayonnement, un stress oxydatif, des dommages à l'ADN, un raccourcissement des télomères, une inflammation, l'usage du tabac et toute combinaison de ceux-ci.

13. Composition pour utilisation selon la revendication 12, dans laquelle ledit rayonnement comprend un rayonnement UV, un rayonnement de lumière visible, un rayonnement infrarouge ou toute combinaison de ceux-ci, et facultativement dans laquelle ledit rayonnement UV est un rayonnement UVA, UVB, UVC ou toute combinaison de ceux-ci.
